(19) Europäisches Patentamt
European Patent Office
Office européen des brevets

(11) **EP 4 640 310 A1**

(12) **EUROPEAN PATENT APPLICATION**
published in accordance with Art. 153(4) EPC

(43) Date of publication:
**29.10.2025 Bulletin 2025/44**

(21) Application number: **23907031.1**

(22) Date of filing: **19.12.2023**

(51) International Patent Classification (IPC):
**B01J 23/10** (2006.01)     **C07B 35/04** (2006.01)
**C07C 2/84** (2006.01)      **C07C 11/04** (2006.01)
**C07B 61/00** (2006.01)

(52) Cooperative Patent Classification (CPC):
**B01J 23/10; C07B 35/04; C07C 2/84; C07C 11/04;**
**C07B 61/00; Y02P 20/52**

(86) International application number:
**PCT/JP2023/045471**

(87) International publication number:
**WO 2024/135671 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.12.2022 JP 2022206233**

(71) Applicant: **Niterra Co., Ltd.
Nagoya-shi, Aichi 461-0005 (JP)**

(72) Inventors:
• **NAKAMURA, Yosuke
Nagoya-shi, Aichi 461-0005 (JP)**

• **WATANABE, Shuntaro
Nagoya-shi, Aichi 461-0005 (JP)**
• **KUROSAWA, Kazuhiro
Nagoya-shi, Aichi 461-0005 (JP)**
• **BAN, Asami
Nagoya-shi, Aichi 461-0005 (JP)**
• **KOZUKA, Hisashi
Nagoya-shi, Aichi 461-0005 (JP)**

(74) Representative: **Grünecker Patent- und
Rechtsanwälte
PartG mbB
Leopoldstraße 4
80802 München (DE)**

(54) **DEHYDROGENATION REACTION CATALYST**

(57)    This dehydrogenation reaction catalyst comprises: a main phase configured from a perovskite oxide expressed by general formula $(A_{1-x}A'_x)(Zr_{1-y-z}B_yB'_z)O_{3-\delta}$ (where A is at least one element selected from alkali earth metals, A' is at least one element from among La and Y, B is at least one element from among Ti and Ce, B' is at least one element from among Y, Sc, Yb, Al, In, and Nd, $0 \le x \le 0.4$, $0.3 \le (1 - z) \le 1$, $0 \le y$, $0 < (1 - y - z)$, and $\delta$ represents an oxygen deficit); and an auxiliary phase configured from at least one from among three composite oxides expressed by general formula $AB'_2O_4$, $A_2B'_2O_5$, or $A_3B'_4O_9$.

FIG. 8

**Description**

TECHNICAL FIELD

The present disclosure relates to a catalyst used in dehydrogenation reaction (hereinafter referred to as a "dehydrogenation reaction catalyst").

BACKGROUND ART

Hitherto, various catalysts have been proposed as those used in methane oxidative coupling for producing $C \geq 2$ hydrocarbons such as ethylene from methane (see, for example, Patent Literatures 1 and 2, and Non-Patent Literature 1). Patent Literature 1 discloses a samarium oxide-on-alkali metal halide catalyst as a methane oxidative coupling catalyst, and that such a catalyst tolerates in a durability test under high temperature conditions (750°C).

CITATION LIST

PATENT LITERATURES

Patent Literature 1: JP1996-010620A
Patent Literature 2: JP1986-282323A

NON-PATENT LITERATURES

Non-Patent Literature 1: Seoyeon Lim, Jae-Wook Choi, Dong Jin Suh, Kwang Ho Song, Hyung Chul Ham, Jeong-Myeong Ha, "Combined experimental and density functional theory (DFT) studies on the catalyst design for the oxidative coupling of methane", Journal of Catalysis, Volume 375, July 2019, Pages 478-492

SUMMARY OF INVENTION

TECHNICAL PROBLEM

Methane oxidative coupling reaction is an exothermic reaction. Thus, as the reaction proceeds, the temperature of the catalyst may steeply rise. For example, when hydrocarbons are produced in large-scale production facilities (i.e., plants) via methane oxidative coupling reaction, the rise in temperature of the catalyst due to rapid progress in the exothermic reaction may be greater. Then, when the temperature of the catalyst rises considerably, a disadvantageous state (e.g., deactivation) of the methane oxidative coupling catalyst may occur. Under such circumstances, there is demand for further improvement in heat resistance of the methane oxidative coupling catalyst. Methane oxidative coupling reaction is a type of dehydrogenation reaction. However, not only methane oxidative coupling reaction, but also other dehydrogenation reactions are generally exothermic reactions. Thus, as the reaction proceeds, the temperature of the catalyst may rise steeply. Accordingly, the aforementioned problem in heat resistance of the catalyst is common among dehydrogenation reaction catalysts.

However, when the related techniques disclosed in Patent Literatures 1 and 2 and Non-Patent Literature 1 are employed, a technique of enhancing heat resistance of a dehydrogenation reaction catalyst is to be further improved. Also, when the related techniques disclosed in Patent Literatures 1 and 2 and Non-Patent Literature 1 are employed, a technique of enhancing a catalytic activity of a dehydrogenation reaction catalyst is to be further improved. In addition, when the related techniques disclosed in Patent Literatures 1 and 2 and Non-Patent Literature 1 are employed, a technique of enhancing a catalytic activity of a dehydrogenation reaction catalyst, particularly a catalytic activity for enhancing dehydrogenation reaction at low temperature, is to be further improved.

SOLUTION TO PROBLEM

The present disclosure can be carried out as the following aspects.

(1) In one aspect of the present disclosure, a dehydrogenation reaction catalyst is provided. The dehydrogenation reaction catalyst includes a primary phase formed of a perovskite-type oxide represented by a general formula $(A_{1-x}A'_x)(Zr_{1-y-z}B_yB'_z)O_{3-\delta}$ (wherein A represents at least one element selected from alkaline earth metals; A' represents at least one element of lanthanum (La) and yttrium (Y); B represents at least one element of titanium (Ti) and cerium (Ce); B' represents at least one element selected from among yttrium (Y), scandium (Sc), ytterbium (Yb), aluminum (Al), indium (In), and neodymium (Nd); relationships: $0 \leq x \leq 0.4$, $0.3 \leq (1-z) \leq 1$, $0 \leq y$, and $0 < (1-y-z)$ are satisfied; and $\delta$ represents an oxygen deficiency), and a secondary phase formed of at least one member of three complex oxides represented by general formulas $AB'_2O_4$, $A_2B'_2O_5$, and $A_3B'_4O_9$, respectively (wherein A and B' are the same elements as A and B' forming the perovskite-type oxide). According to the dehydrogenation reaction catalyst of the aspect, the heat resistance of a dehydrogenation reaction catalyst can be enhanced.

(2) In the dehydrogenation reaction catalyst of the above aspect, A may be barium (Ba), B' may be scandium (Sc), and the secondary phase may be formed of a barium scandate (at least one member of three complex oxides represented by formulas $BaSc_2O_4$, $Ba_2Sc_2O_5$, and $Ba_3Sc_4O_9$). According to such a technical feature, the catalytic activity intrinsic to a dehydrogenation reaction catalyst can be further enhanced.

(3) In the dehydrogenation reaction catalyst of the above aspect, the secondary phase may be formed of $Ba_3Sc_4O_9$. According to such a technical feature, a catalyst having high catalytic activity intrinsic to a dehydrogenation reaction catalyst can be easily produced.

(4) In the dehydrogenation reaction catalyst of the above aspect, the primary phase may be formed of $BaZr_{1-z}Sc_zO_{3-\delta}$ (wherein a relationship $0.1 \leq z \leq 0.7$ is satisfied). According to such a technical feature, the catalytic activity intrinsic to a dehydrogenation reaction catalyst can be further enhanced.

(5) In the dehydrogenation reaction catalyst of the above aspect, in a powder X-ray diffraction pattern obtained by a CuK$\alpha$ ray, the perovskite-type oxide forming the aforementioned primary phase may exhibit a first peak having a highest peak intensity within a diffraction angle range $2\theta$ of 29.5 to 30.5°, and the complex oxide forming the aforementioned secondary phase may exhibit a second peak having a highest peak intensity within a diffraction angle range $2\theta$ of 30.6 to 31.0°. The ratio in intensity of the second peak to the first peak may be 0.04 or greater. According to such a technical feature, the catalytic activity intrinsic to a dehydrogenation reaction catalyst can be further enhanced.

(6) In the dehydrogenation reaction catalyst of the above aspect, the aforementioned secondary phase may be formed of any one member of the three complex oxides. According to such a technical feature, a catalyst having high catalytic activity intrinsic to a dehydrogenation reaction catalyst can be easily produced.

(7) The dehydrogenation reaction catalyst of the above aspect may be a methane oxidative coupling catalyst for producing a C≥2 hydrocarbon from methane. According to such a technical feature, the heat resistance of a methane oxidative coupling catalyst can be enhanced.

(8) In another aspect of the present disclosure, there is provided a dehydrogenation reaction catalyst having the following characteristic feature. An O1s orbital spectrum included in a photoelectron spectrum obtained through X-ray photoelectron spectroscopy is split, through a peak separation fitting processing, to a first curve including a first peak attributed to a maximum value of binding energy within a range of 525 to 530 eV, and a second curve including a second peak attributed to a maximum value of binding energy within a range of 530 to 535 eV. When an area of a convex part including the first peak in the first curve is defined as a first peak area, and an area of a convex part including the second peak in the second curve is defined as a second peak area, a ratio of the second peak area to the first peak area is greater than 1. According to the dehydrogenation reaction catalyst of the aspect, adsorption of adsorbable oxygen species on the surface of the dehydrogenation reaction catalyst is facilitated, whereby the catalytic activity intrinsic to a dehydrogenation reaction catalyst can be enhanced.

(9) The dehydrogenation reaction catalyst of the above aspect has oxygen deficiency. According to such a characteristic feature, the amount of adsorbed oxygen species present on the surface of the dehydrogenation reaction catalyst is increased, whereby the catalytic activity intrinsic to a dehydrogenation reaction catalyst can be further enhanced.

(10) In the dehydrogenation reaction catalyst of the above aspect, the primary phase included in the catalyst has a perovskite structure. According to such a technical feature, the catalytic activity intrinsic to a dehydrogenation reaction catalyst can be further enhanced.

(11) In the dehydrogenation reaction catalyst of the above aspect, a specific surface area as determined through the BET technique is 10 m$^2$/g or more. According to such a characteristic feature, the amount of adsorbed oxygen species present on the surface of the dehydrogenation reaction catalyst is increased, whereby the catalytic activity intrinsic to a dehydrogenation reaction catalyst can be further enhanced.

(12) The dehydrogenation reaction catalyst of the above aspect is a methane oxidative coupling catalyst for producing a C≥2 hydrocarbon from methane. According to such a technical feature, catalytic activity of a methane oxidative coupling catalyst can be enhanced.

(13) In another aspect, there is provided a dehydrogenation reaction catalyst having a perovskite structure represented by a general formula $(La_{1-x}M1_x)M2O_{3-\delta}$ (wherein M1 represents at least one element selected from alkaline earth metals; M2 represents at least one element selected from among yttrium (Y), scandium (Sc), ytterbium (Yb), aluminum (Al), indium (In), and gallium (Ga); $\delta$ represents an oxygen deficiency; and a relationship $0 \leq x \leq 0.6$ is satisfied). According to the dehydrogenation reaction catalyst of the aspect, the catalytic activity intrinsic to a dehydrogenation reaction catalyst (i.e., an activity of promoting dehydrogenation reaction at low temperature) can be enhanced.

(14) In the dehydrogenation reaction catalyst of the above aspect, M1 is strontium (Sr) or barium (Ba). According to such a technical feature, the catalytic activity intrinsic to a dehydrogenation reaction catalyst, specifically, a catalytic activity of promoting dehydrogenation reaction at low temperature, can be further enhanced.

(15) The dehydrogenation reaction catalyst of the above aspect is a methane oxidative coupling catalyst for producing a C≥2 hydrocarbon from methane. According to such a technical feature, the catalytic activity of the methane oxidative coupling catalyst at relatively low temperature can be enhanced.

The present disclosure can be realized in various aspects other than the aforementioned aspects. For example, the present disclosure can be realized as a method for producing a dehydrogenation reaction catalyst, a device accommodating a dehydrogenation reaction catalyst, or an ethylene production device accommodating a methane oxidative coupling catalyst.

BRIEF DESCRIPTION OF DRAWINGS

[FIG. 1] A graph showing a phase diagram of oxides containing barium and scandium.
[FIG. 2] First scheme showing an example of reaction proceeding on a dehydrogenation reaction catalyst.
[FIG. 3] Second scheme showing an example of reaction proceeding on a dehydrogenation reaction catalyst.
[FIG. 4] Representation of various dehydrogenation reactions which the dehydrogenation reaction catalyst can promote.
[FIG. 5] First table showing characteristics of dehydrogenation reaction catalysts of a first embodiment.
[FIG. 6] Second table showing characteristics of dehydrogenation reaction catalysts of the first embodiment.
[FIG. 7] XRD chart of dehydrogenation reaction catalysts of the first embodiment.
[FIG. 8] XRD chart of dehydrogenation reaction catalysts of the first embodiment with some descriptions.
[FIG. 9] First table showing a $C_2$ yield obtained by dehydrogenation reaction catalysts of the first embodiment.
[FIG. 10] Table showing a variation in $C_2$ yield obtained by dehydrogenation reaction catalysts of the first embodiment vs. temperature.
[FIG. 11] Second table showing a $C_2$ yield obtained by dehydrogenation reaction catalysts of the first embodiment.
[FIG. 12] First SEM image of a dehydrogenation reaction catalyst of the first embodiment.
[FIG. 13] Second SEM image of a dehydrogenation reaction catalyst of the first embodiment.
[FIG. 14] Table showing specific surface areas of dehydrogenation reaction catalysts of the first embodiment.
[FIG. 15] First XPS spectrum of dehydrogenation reaction catalysts of the first embodiment.
[FIG. 16] Second XPS spectrum of dehydrogenation reaction catalysts of the first embodiment.
[FIG. 17] Third table showing a $C_2$ yield obtained by dehydrogenation reaction catalysts of the first embodiment.
[FIG. 18] Graph showing calculation of relevant ratios in XPS.
[FIG. 19] Fourth table showing a $C_2$ yield obtained by dehydrogenation reaction catalysts of the first embodiment.
[FIG. 20] Fifth table showing a $C_2$ yield obtained by dehydrogenation reaction catalysts of the first embodiment.
[FIG. 21] Sixth table showing a $C_2$ yield obtained by dehydrogenation reaction catalysts of the second embodiment.

DESCRIPTION OF EMBODIMENT

<First embodiment>

[Dehydrogenation reaction catalyst]

The dehydrogenation reaction catalyst of the present embodiment is a complex oxide which includes a primary phase having a perovskite-type oxide crystal structure represented by the below-mentioned general formula (1), and a secondary phase containing at least one member of three complex oxides represented by the below-mentioned general formulas (2a) to (2c).

$$(A_{1-x}A'_x)(Zr_{1-y-z}B_yB'_z)O_{3-\delta} \cdots \quad (1)$$

$$AB'_2O_4 \cdots \quad (2a)$$

$$A_2B'_2O_5 \cdots \quad (2b)$$

$$A_3B'_4O_9 \cdots \qquad (2c)$$

In the aforementioned formula (1), A represents at least one element selected from alkaline earth metals; A' represents at least one element of lanthanum (La) and yttrium (Y); B represents at least one element of titanium (Ti) and cerium (Ce); B' represents at least one element selected from among yttrium (Y), scandium (Sc), ytterbium (Yb), aluminum (Al), indium (In), and neodymium (Nd). In formula (1), x, y, and z satisfy relationships represented by the following formulas (3a) to (3d), and $\delta$ represents an oxygen deficiency to achieve electrical neutrality, for example, $0 \leq \delta \leq 0.5$.

$$0 \leq x \leq 0.4 \quad \cdots \quad (3a)$$

$$0.3 \leq (1-z) \leq 1 \quad \cdots \quad (3b)$$

$$0 \leq y \quad \cdots \quad (3c)$$

$$0 < (1-y-z) \quad \cdots \quad (3d)$$

In the aforementioned formulas (2a) to (2c), A and B' are the same elements as A and B' forming the perovskite-type oxide.

Also, the dehydrogenation reaction catalyst of the present embodiment has the following characteristic feature. That is, an O1s orbital spectrum included in a photoelectron spectrum obtained through X-ray photoelectron spectroscopy is split, through a peak separation fitting processing, to a first curve a first peak attributed to a maximum value of binding energy within a range of 525 to 530 eV, and a second curve including a second peak attributed to a maximum value of binding energy within a range of 530 to 535 eV. When an area of a convex part including the first peak in the first curve is defined as a first peak area, and an area of a convex part including the second peak in the second curve is defined as a second peak area, a ratio of the second peak area to the first peak area is greater than 1. In the O1s orbital spectrum included in a photoelectron spectrum obtained through X-ray photoelectron spectroscopy, the first peak area is attributable to in-lattice oxygen species present at the surface of the dehydrogenation reaction catalyst, and the second peak area is attributable to adsorbed oxygen species present on the surface of the dehydrogenation reaction catalyst. Thus, a ratio of the second peak area to the first peak area of greater than 1 means that a large amount of activated adsorbed oxygen species are present in the dehydrogenation reaction catalyst. In such a case, dehydrogenation reaction is promoted. The "first peak area" and "second peak area" will be described in detail below.

The dehydrogenation reaction catalyst having a ratio of the second peak area to the first peak area of greater than 1 preferably has oxygen deficiency. Specifically, in the case of a dehydrogenation reaction catalyst represented by a compositional formula $Ba(Zr_{1-x}Y_x)O_{3-\delta}$, the catalyst readily has oxygen deficiency for compensating electric charge when x is larger. In this case, the amount of activated adsorbed oxygen species present on the surface of the dehydrogenation reaction catalyst can increase. From another aspect, the dehydrogenation reaction catalyst having a ratio of the second peak area to the first peak area of greater than 1 preferably has a specific surface area as determined through the BET technique is 10 $m^2/g$ or more. In this case, the amount of activated adsorbed oxygen species present on the surface of the dehydrogenation reaction catalyst can increase.

In the dehydrogenation reaction catalyst of the present embodiment, at least the perovskite-type oxide represented by formula (1) and forming the primary phase has an activity intrinsic to dehydrogenation reaction catalyst. As used herein, the term "primary phase" refers to a phase exhibiting 50% or more of the peak intensity in a powder X-ray diffraction pattern obtained with a CuK$\alpha$ ray. In addition to the aforementioned primary phase, the dehydrogenation reaction catalyst of the present embodiment has a secondary phase formed of at least one member of the three complex oxides represented by formulas (2a) to (2c). As a result, the heat resistance of the catalyst is enhanced.

[Perovskite-type oxide forming primary phase]

As described above, in the dehydrogenation reaction catalyst of the present embodiment, at least the perovskite-type oxide represented by formula (1) and forming the primary phase has an activity intrinsic to dehydrogenation reaction catalyst. Thus, the perovskite-type oxide of formula (1) will be described in more detail.

The perovskite-type oxide of formula (1) contains at least one element selected from alkaline earth metals in A sites of the perovskite structure. The alkaline earth metal is preferably an element selected from among barium (Ba), calcium (Ca), and strontium (Sr). Hereinafter, the alkaline earth metal element present in the aforementioned A sites may also be referred to as an "element A." The perovskite-type oxide of formula (1) may further contain at least one element of lanthanum (La) and yttrium (Y) in A sites of the perovskite structure. However, lanthanum (La) and yttrium (Y) are not essential elements. Hereinafter, lanthanum (La) and yttrium (Y) present in the aforementioned A sites may also be referred to as "elements A'." In the dehydrogenation reaction catalyst of the present embodiment, the element A content and the element A' content satisfy the formula (3a) in the aforementioned formula (1).

Also, the perovskite-type oxide of formula (1) contains zirconium (Zr) in B sites of the perovskite structure. The perovskite-type oxide may further contain at least one element of titanium (Ti) and cerium (Ce) in the B sites. However, titanium (Ti) and cerium (Ce) are not essential elements. Hereinafter, titanium (Ti) and cerium (Ce) present in the aforementioned B sites may also be referred to as "elements B." The perovskite-type oxide of formula (1) may further contain at least one element selected from among yttrium (Y), scandium (Sc), ytterbium (Yb), aluminum (Al), indium (In), and neodymium (Nd) in B sites of the perovskite structure. However, at least one element selected from among yttrium (Y), scandium (Sc), ytterbium (Yb), aluminum (Al), indium (In), and neodymium (Nd) are not essential elements. Hereinafter, at least one element selected from among yttrium (Y), scandium (Sc), ytterbium (Yb), aluminum (Al), indium (In), and neodymium (Nd) present in the aforementioned B sites may also be referred to as "elements B'." In the perovskite-type oxide represented of formula (1), the Zr content, the element B content, and the element B' content satisfy the formulas (3b) to (3d) in the aforementioned formula (1).

In the perovskite-type oxide of formula (1), x preferably satisfies the following formula (3e). In such a case, the catalytic activity intrinsic to a dehydrogenation reaction catalyst, specifically, an activity of promoting dehydrogenation reaction, can be further enhanced.

$$0 \leq x \leq 0.2 \quad \cdots \quad (3e)$$

The perovskite-type oxide of formula (1) preferably has a perovskite structure represented by the following general formula (4). In such a case, the catalytic activity intrinsic to a dehydrogenation reaction catalyst, specifically, an activity of promoting dehydrogenation reaction, can be further enhanced.

$$BaZr_{1-z}B'_zO_{3-\delta} \cdots \quad (4)$$

(wherein B' represents at least one element selected from among yttrium (Y), scandium (Sc), ytterbium (Yb), indium (In), and neodymium (Nd)). Particularly preferably, B' is scandium (Sc); i.e., the perovskite-type oxide of formula (4) is $BaZr_{1-z}Sc_zO_{3-\delta}$ (wherein the relationship $0.1 \leq z \leq 0.7$ is satisfied). In such a case, the catalytic activity intrinsic to a dehydrogenation reaction catalyst, specifically, an activity of promoting dehydrogenation reaction, can be further enhanced.

In addition to the catalytic activity intrinsic to a dehydrogenation reaction catalyst, specifically, an activity of promoting dehydrogenation reaction, the perovskite-type oxide of formula (1) has at least one of proton conductivity, electron conductivity, and hole conductivity.

[Complex oxide forming secondary phase]

As described above, the dehydrogenation reaction catalyst of the present embodiment has a secondary phase formed of at least one member of the three complex oxides represented by formulas (2a) to (2c). By virtue of such a structural characteristic, an enhanced heat resistance is achieved. More specifically, even when the dehydrogenation reaction catalyst is used under high temperature conditions (e.g., 800°C or higher), a drop in catalytic activity intrinsic to a dehydrogenation reaction catalyst, specifically, an activity of promoting dehydrogenation reaction, can be suppressed. Notably, the secondary phase is preferably formed of any one member of the three complex oxides represented by formulas (2a) to (2c).

In formulas (2a) to (2c), A and B' are the same elements as A and B' forming the perovskite-type oxide of formula (1) forming the primary phase. As described hereinbelow, such complex oxides of the secondary phase can be formed simultaneously with a perovskite-type oxide of the primary phase in production of a dehydrogenation reaction catalyst, by mixing raw material powders of the perovskite-type oxide forming the primary phase, and firing the mixture at relatively high temperature (about 1,300 to about 1,600°C). Among the complex oxides represented by formulas (2a) to (2c), the species of the complex oxide forming the secondary phase is determined by the types of elements present in A sites and B sites of the perovskite-type oxide of formula (1). Also, among the above complex oxides, the species of the complex oxide forming the secondary phase may be varied by the proportions of mixing raw material powders of the perovskite-type oxide forming the primary phase in production of the dehydrogenation reaction catalyst.

In the dehydrogenation reaction catalyst of the present embodiment, preferably, , element A is barium (Ba), and element B' is scandium (Sc), in formula (1), and the secondary phase is formed of barium scandate; i.e., at least one member of the three complex oxides: $BaSc_2O_4$, $Ba_2Sc_2O_5$, and $Ba_3Sc_4O_9$. Particularly preferably, the secondary phase is

formed of $Ba_3Sc_4O_9$.

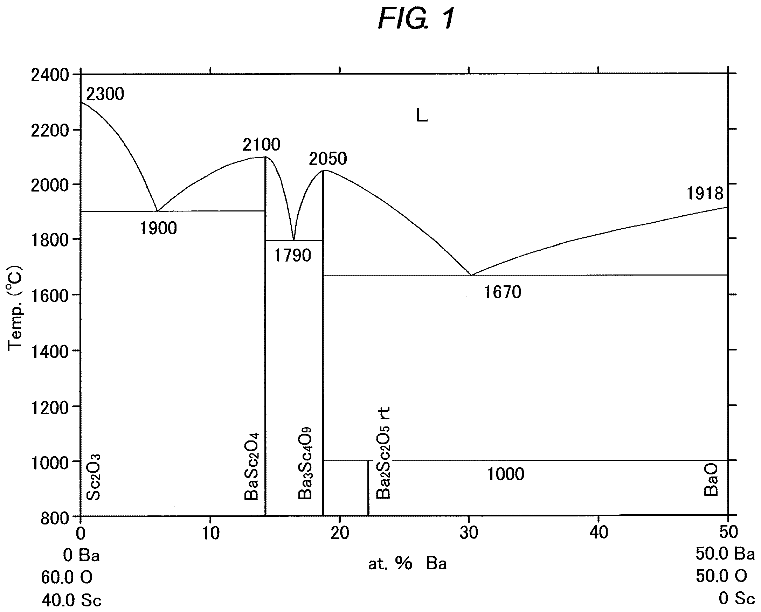

FIG. 1 is a graph showing an exemplary phase diagram of oxides containing barium (Ba) and scandium (Sc) (source: Kovba L. M., Lykova L.N., Paromova M. V., Kalinina T. A. (1981). X-Ray and thermographic investigation of compounds in the BaO-$Sc_2O_3$ system. Dokl. Akad. Nauk SSSR, 260, 924-927). As shown in FIG. 1, it is understood that the three complex oxides: $BaSc_2O_4$, $Ba_2Sc_2O_5$, and $Ba_3Sc_4O_9$, can be formed as complex oxides each containing barium (Ba) and scandium (Sc). It is also understood, in the case where an alkaline earth metal other than barium (Ba) is used as element A, and any of the aforementioned elements other than scandium (Sc) is used as element B' in the complex oxides shown in the phase diagram, the three complex oxides represented by formulas (2a) to (2c) can be formed.

In a powder X-ray diffraction pattern obtained by a CuKα ray, when the highest peak attributed to the perovskite-type oxide forming the primary phase of the dehydrogenation reaction catalyst of the present embodiment is defined as a first peak, and the highest peak attributed to the complex oxide forming the secondary phase is defined as a second peak, the ratio in intensity of the second peak to the first peak is preferably 0.02 or greater, more preferably 0.03 or greater, still more preferably 0.04 or greater. Thus, by securing the ratio of the complex oxide forming the secondary phase, the heat resistance of the dehydrogenation reaction catalyst can be enhanced in a more easy manner. For example, when the perovskite-type oxide forming the primary phase is $BaZr_{1-z}Sc_zO_{3-\delta}$, and the complex oxide forming the secondary phase is $Ba_3Sc_4O_9$, the first peak emerges within a diffraction angle range 2θ of 29.5 to 30.5°, and the second peak emerges within a diffraction angle range 2θ of 30.6 to 31.0°, in a powder X-ray diffraction pattern obtained by a CuKα ray.

The dehydrogenation reaction catalyst of the present embodiment having the aforementioned characteristic features; i.e., including a primary phase having a perovskite-type oxide crystal structure represented by the aforementioned formula (1), and a secondary phase containing at least one member of the three complex oxides represented by the aforementioned formulas (2a) to (2c). Thus, the catalytic activity intrinsic to a dehydrogenation reaction catalyst (i.e., an activity of promoting dehydrogenation reaction to release hydrogen from a compound), and the heat resistance of the dehydrogenation reaction catalyst can be enhanced. In the dehydrogenation reaction catalyst of the present embodiment, the catalytic activity of promoting dehydrogenation reaction is achieved mainly on the basis of the perovskite-type oxide forming the primary phase. In such a dehydrogenation reaction catalyst, when the secondary phase containing at least one member of the three complex oxides represented by formulas (2a) to (2c) is added, a drop in catalytic activity of promoting dehydrogenation reaction can be suppressed, even in the case where the dehydrogenation reaction catalyst is used in higher temperature conditions.

The dehydrogenation reaction catalyst of the present embodiment can promote various dehydrogenation reactions for releasing hydrogen from a compound. Examples of the dehydrogenation reaction include a methane oxidative coupling (i.e., oxidative coupling of methane: OCM) reaction for producing a C≥2 hydrocarbon from methane, and various dehydrogenation reactions for forming hydrogen from a variety of hydrocarbon-based compounds including hydrocarbon and alcohol. Specific examples thereof include steam reformation reaction (i.e., forming hydrogen from the aforementioned hydrocarbon-based compound and steam), partial oxidation reaction (i.e., forming hydrogen from the aforementioned hydrocarbon-based compound), and shift reaction (i.e., forming carbon dioxide and hydrogen by use of carbon monoxide and steam generated with hydrogen in the aforementioned partial oxidation reaction).

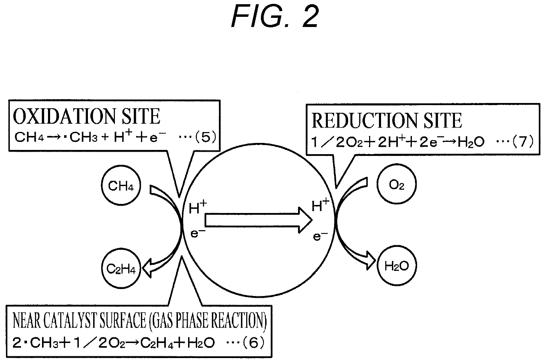

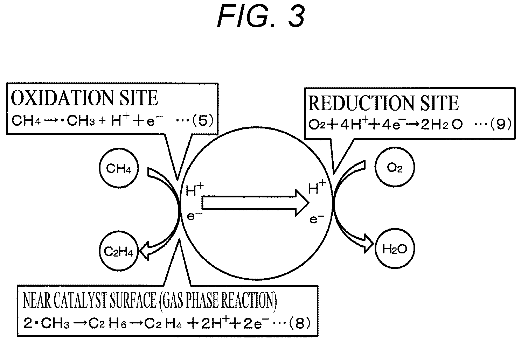

FIG. 2 is a first scheme showing an example of reaction proceeding on a dehydrogenation reaction catalyst, and FIG. 3 is a second scheme showing an example of reaction proceeding on a dehydrogenation reaction catalyst. Collectively, FIGs. 2 and 3 are schemes each showing an example of methane oxidative coupling reaction proceeding on a methane oxidative coupling catalyst, in the case where the dehydrogenation reaction catalyst of the present embodiment corresponds to the methane oxidative coupling catalyst. In proceeding of a dehydrogenation reaction for releasing hydrogen from a compound, a covalent bond between a hydrogen atom and another atom (e.g., a carbon atom forming the compound) is generally broken. The dehydrogenation reaction catalyst of the present embodiment exhibits high activity of promoting dehydrogenation reaction, conceivably because the catalyst promotes the reactions of the aforementioned modes. In methane oxidative coupling reaction, conceivably, a reaction for generating methyl radicals from methane (see the following formula (5)), and reactions (see the following formulas (6) and (7) in FIG. 2) or reactions (see the following formulas (8) and (9) in FIG. 3) proceed, to thereby form $C_2$ hydrocarbons such as ethylene. The overall reaction for forming ethylene via methane oxidative coupling reaction is represented by the following formula (10).

$$CH_4 \rightarrow \cdot CH_3 + H^+ + e^- \cdots \quad (5)$$

$$2 \cdot CH_3 + 1/2 O_2 \rightarrow C_2H_4 + H_2O \cdots \quad (6)$$

$$1/2 O_2 + 2H^+ + 2e^- \rightarrow H_2O \cdots \quad (7)$$

$$2 \cdot CH_3 \rightarrow C_2H_6 \rightarrow C_2H_4 + 2H^+ + 2e^- \cdots \quad (8)$$

$$O_2 + 4H^+ + 4e^- \rightarrow 2H_2O \cdots \quad (9)$$

$$2CH_4 + O_2 \rightarrow C_2H_4 + 2H_2O \cdots \qquad (10)$$

In the above case, the covalent bond between a carbon atom and a hydrogen atom of methane is considerably stable with a binding energy of 104 kcal/mol. Thus, conceivably, a reaction of generating methyl radicals through dehydrogenation of methane having the aforementioned stable covalent bond generally plays a role of a rate-determining step (i.e., reaction represented by the aforementioned formula (5)) in methane oxidative coupling reaction. Since the dehydrogenation reaction catalyst of the present embodiment exhibits, in at least the primary phase, a high activity of promoting a reaction involving cut of a covalent bond with a hydrogen atom (e.g., the aforementioned reaction of forming methyl radicals from methane), a wide range of dehydrogenation reactions involving cut of a covalent bond with a hydrogen atom (e.g., methane oxidative coupling reaction) can be conceivably promoted. Furthermore, at least the primary phase of the dehydrogenation reaction catalyst of the present embodiment is formed of the aforementioned proton conductor, whereby a hydrogen in a hydrogen-containing molecule is protonated and transferred. Thus, conceivably, the dehydrogenation reaction catalyst exhibits a high activity of promoting a reaction involving cut of a covalent bond with a hydrogen atom, whereby a wide range of dehydrogenation reactions involving cut of a covalent bond with a hydrogen atom (e.g., methane oxidative coupling reaction) can be promoted.

In FIGs. 2 and 3, a site where a reaction of formula (5) for forming methyl radicals from methane proceeds is denoted as an "oxidation site." FIGs. 2 and 3 show proceeding of a reaction of formula (6) or (8) for forming ethylene from methyl radical at the oxidation site. Also, in FIGs. 2 and 3, a site where a reaction of formula (7) or (9) for forming water by use of protons and electrons generated with methyl radicals according to formula (5) proceeds is denoted as a "reduction site." Thus, in proceeding of a reaction involving the dehydrogenation reaction in the dehydrogenation reaction catalyst of the present embodiment, transfer of protons and electrons generally is involved. Therefore, in order to enhance catalytic activity, the dehydrogenation reaction catalyst preferably has high activity of promoting the aforementioned reaction of formula (5), which is rate-determining step, and high proton conductivity and electron conductivity (including hole conductivity). Specifically, proton conductivity and proton transport number, or electron conductivity (including hole conductivity) and electron transport number (including hole transport number) are preferably higher. In addition, the entirety of the dehydrogenation reaction catalyst preferably has a higher whole conductivity, which is a sum of proton conductivity and electron conductivity (including hole conductivity).

From such a viewpoint, the whole conductivity of the primary phase of the dehydrogenation reaction catalyst of the present embodiment is preferably, for example, $1.0 \times 10^{-5}$ S/cm or higher, and the proton conductivity of the primary phase is preferably $1.0 \times 10^{-4}$ S/cm or higher. The proton transport number of the primary phase is preferably 0.01 or greater. The sum of the electron transport number and the hole transport number of the primary phase is preferably 0.01 or greater. In the primary phase, the proton transport number and the sum of the electron transport number and the hole transport number are each preferably 0.10 or greater. In contrast, when electron conductivity (including hole conductivity) excessively increases, in the case of, for example, methane oxidative coupling reaction, peroxidation of methane (i.e., complete oxidation reaction for forming carbon dioxide and water from methane) easily proceeds, whereby a reaction for forming ethylene from methane may be suppressed. Thus, the whole conductivity of the primary phase of the dehydrogenation reaction catalyst is preferably $1.0 \times 10^{-1}$ S/cm or lower, and the sum of the electron transport number and the hole transport number of the primary phase is preferably 0.95 or smaller. When the perovskite-type oxide forming the primary phase of the dehydrogenation reaction catalyst satisfies the aforementioned formulas (1) and (3a) to (3d), the above numerical ranges are easily satisfied.

[Other dehydrogenation reactions]

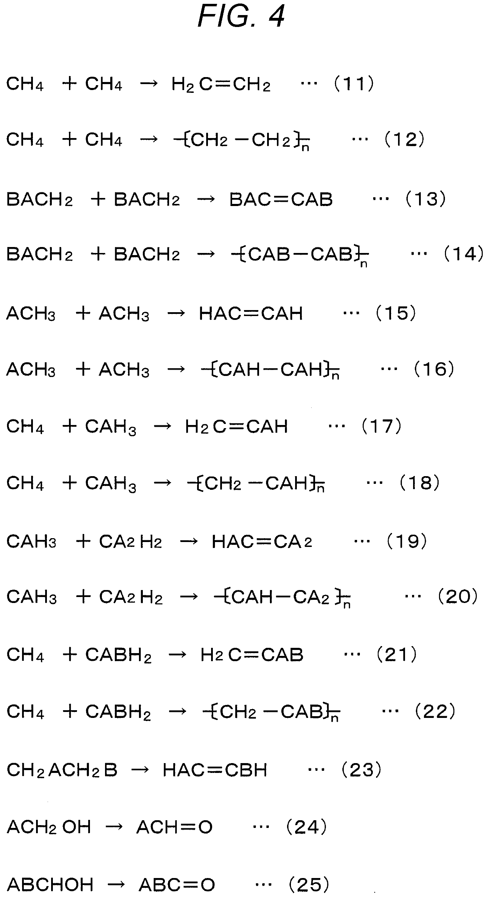

FIG. 4 is a representation of various examples of dehydrogenation reaction which the dehydrogenation reaction catalyst of the present embodiment can promote. The dehydrogenation reaction catalyst of the present embodiment can promote various reactions depending on the raw materials (reactants) employed. The formulas (11) to (22) in FIG. 4 each represent a reaction in which reactants including at least one of methane and a methane derivative (hereinafter may also be collectively referred to as "METHANE") are bound. Such reaction may also be called "METHANE oxidative coupling reaction." Also, the dehydrogenation reaction catalyst of the present embodiment for promoting METHANE oxidative coupling reaction may also be referred to as a "METHANE oxidative coupling catalyst." In formulas (13) to (22) in FIG. 4, each of the references A and B present in a methane derivative refers to an atom or a group serving as a substituent for hydrogen of a methane molecule, and n is an integer of 2 or greater. In each of the methane derivatives shown in FIG. 4,, one or two hydrogen atoms of the methane molecule are substituted by any of the aforementioned substituents. In the case where one molecule has substituents A and B, A and B may be the substituents identical to or different from each other. Examples of the substituent A or B include a halogen element, a hydroxy group (-OH), and a phenyl group ($-C_6H_5$).

In FIG. 4, similar to formula (10), formula (11) represents a reaction for forming ethylene from methane. Formula (12) represents a reaction involving a further polymerization reaction to form polyethylene. Differing from formula (10), formulas (11) to (22) represent only a change in methane and a methane derivative among the molecules involved in the relevant reaction.

Formulas (11) and (12) represent a reaction involving methane as a reactant. Formulas (13) and (14) represent a reaction involving a methane derivative represented by $BACH_2$ as a reactant. Formulas (15) and (16) represent a reaction involving a methane derivative represented by $ACH_3$ as a reactant. Formulas (17) and (18) represent a reaction involving a methane derivative represented by $ACH_3$ and methane as reactants. Formulas (19) and (20) represent a reaction involving a methane derivative represented by $ACH_3$ and a methane derivative represented by $CA_2H_2$ as reactants. Formulas (21) and (22) represent a reaction involving a methane derivative represented by $CABH_2$ and methane as reactants.

Formulas (11), (13), (15), (17), (19), and (21) each represent a reaction for forming a C2 hydrocarbon (ethylene) or an ethylene derivative (hereinafter may also be collectively referred to as a "$C_2$ HYDROCARBON") by use of a reactant selected from a C1 hydrocarbon (methane) and a methane derivative (hereinafter may also be collectively referred to as a "$C_1$ HYDROCARBON"). Formulas (12), (14), (16), (18), (20), and (22) each represent a reaction for forming a polymer by use of a reactant selected from the $C_1$ HYDROCARBONs. In formation of a polymer, after formation of a $C_2$ HYDRO-CARBON from a $C_1$ HYDROCARBON, $C_2$ HYDROCARBON molecules may be further polymerized. Alternatively, a $C_1$ HYDROCARBON may be successively polymerized at an end of a molecule, to thereby form a polymer. These polymerization reactions may proceed in parallel.

The reactions shown in FIG. 4 will be described in more detail. In formula (14), when each of the substituents A and B is a fluorine atom (F), polytetrafluoroethylene (PTFE) is yielded as a product. In formula (18), when the substituent A is a chlorine atom (Cl), poly(vinyl chloride) (PVC) is yielded as a product. When the substituent A is a hydroxy group (-OH), poly(vinyl alcohol) (PVOH) is yielded as a product. When the substituent A is a phenyl group ($-C_6H_5$), polystyrene (PS) is yielded as a product. In formula (22), when each of the substituents A and B is a fluorine atom (F), poly(vinylidene fluoride (PVDF) is yielded as a product. When each of the substituents A and B is a chlorine atom (Cl), poly(vinylidene chloride) (PVDC) is yielded as a product. In formula (22), when the substituent A is a methyl group ($-CH_3$), and the substituent B is a methoxycarbonyl group ($-COOCH_3$), poly(methyl methacrylate) (PMMA) is yielded as a product.

In FIG. 4, specific METHANE oxidative coupling reactions are shown. However, the dehydrogenation reaction catalyst of the present embodiment may be used for promoting a dehydrogenation reaction other than the METHANE oxidative coupling reactions. Examples of the dehydrogenation reaction which the dehydrogenation reaction catalyst of the present embodiment can promote include a reaction in which reactants including at least one of an alkane and an alkane derivative (hereinafter may also be collectively referred to as "ALKANE") are bound. Such a reaction may also be referred to an "ALKANE oxidative coupling reaction" collectively with the aforementioned METHANE oxidative coupling reaction. The dehydrogenation reaction catalyst of the present embodiment which catalyst promotes the ALKANE oxidative coupling reaction may also be referred to as an "ALKANE oxidative coupling catalyst." The number of carbon atoms of the ALKANE serving as a reactant of the ALKANE oxidative coupling reaction which the dehydrogenation reaction catalyst of the present embodiment promotes may be, for example, 1 to 10, and is preferably 1 to 5, more preferably 1 or 2. Notably, the alkane derivative which is subjected to the ALKANE oxidative coupling reaction is derived by substituting hydrogen present in an alkane molecule with any of the aforementioned substituents, and a covalent bond is present between a hydrogen atom and a carbon atom of the dehydration target.

The dehydrogenation reaction catalyst of the present embodiment may also be used for promoting a dehydrogenation reaction other than ALKANE oxidative coupling reaction. Examples of another dehydrogenation reaction which the dehydrogenation reaction catalyst of the present embodiment promotes include a dehydrogenation reaction of ALKANE as exemplified in formula (23) of FIG. 4, and a dehydrogenation reaction of an alcohol or an alcohol derivative (hereinafter may also be collectively referred to as "ALCOHOL") as exemplified in formulas (24) and (25). In formulas (23) to (25), each of A and B represents hydrogen, or an atom or a group substituting for hydrogen, similar to the cases of formulas (13) to (22). Formula (23) represents a reaction for forming an alkene or an alkene derivative from ALKANE. Formula (24) represents a reaction for forming an aldehyde from ALCOHOL. Formula (25) represents a reaction for forming a ketone from ALCOHOL. As described above, the dehydrogenation reaction catalyst of the present embodiment may be used as a catalyst for promoting various dehydrogenation reactions.

[Dehydrogenation reaction catalyst production method]

The dehydrogenation reaction catalyst of the present embodiment may be produced through, for example, a solid-phase reaction method. In a specific procedure, powders of metal oxides or metal carbonates serving as raw materials of the primary phase represented by formula (1) are mixed together with a solvent such as ethanol by means of a ball mill or the like. Then, the mixture is dried to remove the solvent and fired. When firing is conducted at relatively high temperature (about 1,300 to 1,600°C), there can be yielded a complex oxide which include the primary phase represented by formula (1) and a secondary phase containing at least one member of the three complex oxides represented by the below-mentioned general formulas (2a) to (2c).

As a method for producing the complex oxide, there have been known a variety of methods such as a solid-phase reaction method, a complex polymerization method, a co-precipitation method, and a sol-gel method. In particular, a complex polymerization method is known as a suitable catalyst production method, since raw materials can be mixed at a molecular level. However, when such a complex polymerization method is employed, the structure of the product readily varies by heat. When firing is conducted at the aforementioned high temperature, variation such as an increase in grain size readily occurs. As a result, catalytic performance may be deteriorated. In contrast, the solid-phase reaction method less provides structural variation possibly by heat. Even when firing is conducted at the aforementioned high temperature, a drop in catalytic performance can be suppressed. Further, in the case where the dehydrogenation reaction catalyst is produced through the solid-phase reaction method involving firing at the aforementioned high temperature, the structure of the complex oxide is stabilized even under high temperature firing conditions. Thus, when the thus-obtained dehydrogenation reaction catalyst is employed at a temperature lower than the firing temperature (in proceeding of dehydrogenation reaction), progress of deterioration such as structural variation, including volatilization of a constituent element, can be suppressed. As a result, for example, in the case where the temperature of the catalyst steeply rises in the course of exothermic reaction including dehydrogenation reaction, an undesired phenomenon such as deactivation of the dehydrogenation reaction catalyst can be suppressed, whereby heat resistance and durability of the dehydrogenation reaction catalyst can be enhanced.

<Example 1>

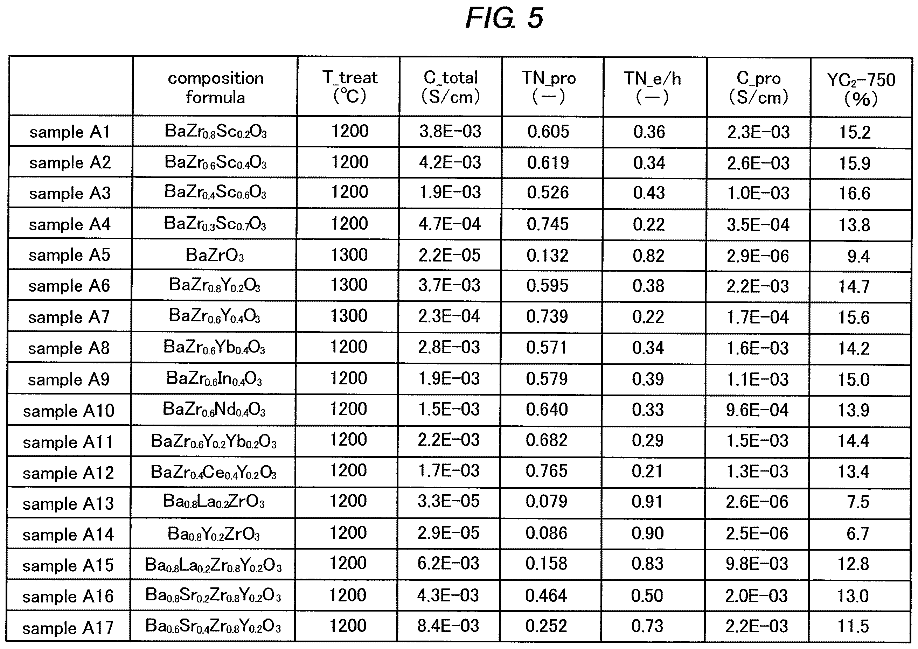

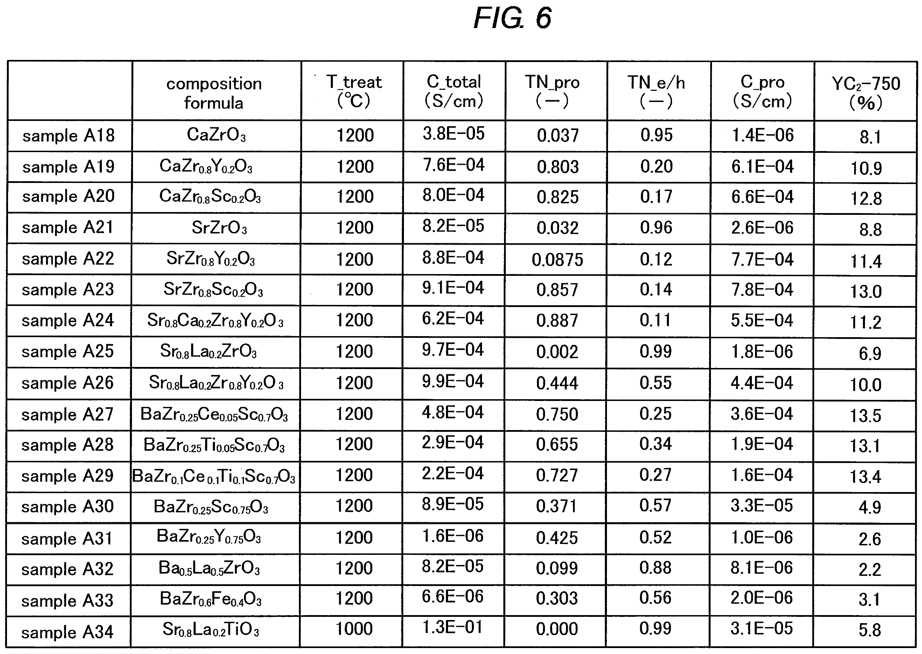

FIG. 5 is a first table showing characteristics of dehydrogenation reaction catalysts of a present embodiment. FIG. 6 is a second table showing characteristics of dehydrogenation reaction catalysts of the present embodiment. FIGs. 5 and 6 show performances investigated with respect to the prepared 34 complex oxides; i.e., samples A1 to A34. In Example 1, the performance of a dehydrogenation reaction catalyst was evaluated as that of a methane oxidative coupling catalyst. Among samples A1 to A34, samples A1 to A29 each correspond to the primary phase of the dehydrogenation reaction catalyst of the aforementioned embodiment, and to a perovskite-type oxide satisfying any of the aforementioned formulas (1) and (3a) to (3d). Samples A30 to A34 each correspond to a complex oxide not satisfying at least any of the formulas (1) and (3a) to (3d). These complex oxides of samples A1 to A34 were produced through a complex polymerization method. Notably, in the following description and drawings, the number of oxygen atoms in any formula may be denoted simply as "$O_3$" in some cases. However, the denotation is equivalent to "$O_{3-\delta}$" and does not mean only the case in which $\delta$ is strictly 0. Next, the composition, production method, evaluation method, and results of performance evaluation with respect to the samples will be described.

[Production of sample A1]

A catalyst ($BaZr_{0.8}Sc_{0.2}O_3$) of sample A1 was prepared through a complex polymerization method. As raw material powders, barium nitrate (product of FUJIFILM Wako Pure Chemical Corporation), zirconyl nitrate (product of FUJIFILM Wako Pure Chemical Corporation), and scandium nitrate (product of Alfa Aesar) were used. These raw material powders were weighed so that the proportion of metal elements were adjusted to establish the composition of a compositional formula $BaZr_{0.8}Sc_{0.2}O_3$. To the mixture of the above raw material powders, aqueous citric acid and propylene glycol were added so that the proportions: metal element : citric acid : propylene glycol were adjusted to 1:3:3 (by mole). The resultant mixture was agitated and sufficiently mixed at 80°C for 1 hour. The resultant solution was slowly heated to 300°C, to thereby yield a polymer in which the metals were dispersed. The thus-obtained polymer was heated at 400°C for 2 hours, and the formed carbonized product was pulverized by means of an agate mortar, to thereby yield a precursor of a catalyst powder. The thus-obtained catalyst powder precursor was subjected to a heat treatment at 1,200°C ("T_treat," in FIGs. 5 and 6) for 6 hours, to thereby yield a powder-form dehydrogenation reaction catalyst as sample A1.

In sample A1 and the below-mentioned samples A2 to A34, whether or not the obtained powder-form methane oxidative coupling catalyst had a target composition was confirmed through a powder XRD method. More specifically, achievement of a perovskite structure, and absence of peaks attributed to a raw material powder or a single oxide (i.e., an oxide containing only one metal element other than oxygen) or a carbonate salt originating from the raw material powder were confirmed the results of powder XRD analysis. In sample A1 and the below-mentioned samples A2 to A34, the specific surface area of the obtained powder-form methane oxidative coupling catalyst was determined through a gas adsorption method. In all cases, the specific surface area was found to fall within a range of 5 $m^2/g$ to 10 $m^2/g$.

[Production of samples A2 to A34]

Powder-form dehydrogenation reaction catalysts of samples A2 to A34 were produced in a manner similar to production of sample A1, except that the type and weighing amount of a catalyst raw material powder were changed to attain the compositional proportions of the compositional formulas of samples A2 to A34 shown in FIGs. 5 and 6, and that the temperature of the heat treatment of a catalyst powder precursor was changed to the heat treatment temperatures shown in FIGs. 5 and 6 ("T_treat," in FIGs. 5 and 6). In order to add, as constituent elements, barium, zirconium, scandium, yttrium, ytterbium, indium, neodymium, cerium, lanthanum, strontium, calcium, iron, and titanium, there were used the following raw material powders: barium nitrate (product of FUJIFILM Wako Pure Chemical Corporation), zirconyl nitrate (product of FUJIFILM Wako Pure Chemical Corporation), scandium nitrate (product of Alfa Aesar), yttrium nitrate (product of Sigma Aldrich), ytterbium nitrate (product of Sigma Aldrich), indium nitrate (product of FUJIFILM Wako Pure Chemical Corporation), neodymium nitrate (product of FUJIFILM Wako Pure Chemical Corporation), cerium nitrate (product of FUJIFILM Wako Pure Chemical Corporation), lanthanum nitrate (product of FUJIFILM Wako Pure Chemical Corporation), strontium nitrate (product of FUJIFILM Wako Pure Chemical Corporation), calcium nitrate (product of FUJIFILM Wako Pure Chemical Corporation), iron nitrate (product of Sigma Aldrich), and tetra-i-propoxytitanium (product of Kojundo Chemical Laboratory Co., Ltd.).

[Measurement of whole conductivity]

Each of samples A1 to A34 was press-molded to a rectangular parallelepiped piece and baked at 1,600°C for 6 hours for sintering. A platinum wire was wound around four sites of the piece, and a platinum paste (TR-7905, product of Tanaka Kikinzoku Kogyo) was applied thereonto. The piece was fired at 1,000°C for 1 hour, to thereby provide a measurement piece. The measurement piece was placed in a tube-shape electric furnace and heated under a flow of hydrogen moisturized through a bubbler. The whole conductivity was measured through an AC 4-terminal method at 750°C ("C_total," in FIGs. 5 and 6). The relative density of each sintered body employed in whole conductivity measured was determined through the Archimedes method. As a result, all the sintered bodies were found to have a relative density of 96% or higher.

[Measurement of proton transport number and electron/hole transport number, and calculation of proton conductivity]

Each of samples A1 to A34 was press-molded to a disk and baked at 1,600°C for 6 hours for sintering. A platinum paste was applied onto each surface of the disk to a circular coating through screen printing. The disk was fired at 1,000°C for 1 hour, to thereby provide a measurement piece. The apparatus for measuring transport numbers was equipped with two alumina tubes disposed up and down along the common axis. Between the two alumina tubes, a measurement sample was inserted, and gas sealing of the measurement sample with the two alumina tubes was secured. A portion including the measurement sample was placed in an electric furnace, and the transport numbers were measured. More specifically, moisturized hydrogen was caused to flow through a section and the other section isolated by the measurement sample in the two alumina tubes, while the concentration and moisture amount varied between the two sections. Under the conditions, the measurement sample was heated at 750°C by means of the aforementioned electric furnace. Then, electromotive force generated between one surface and the other surface of the measurement sample was measured, to thereby determine proton transport number ("TN_pro," in FIGs. 5 and 6) and electron/hole transport number ("TN_e/h," in FIGs. 5 and 6). Proton conductivity ("C_pro," in FIGs. 5 and 6) was calculated by multiplying the above-measured whole conductivity by proton transport number.

[Determination of $C_2$ yield]

Each of samples A1 to A34 was weighed to take a portion of 0.1 g and placed in an immobilized bed flow reactor. While the reactor was heated at 750°C, a gas mixture containing methane, oxygen, and nitrogen was caused to flow through the reactor at a pressure of 1 atm, a flow rate of 45 cm$^3$/min, and flow proportions $CH_4$:$O_2$:$N_2$ of 3.8:1:4. Under the conditions, a catalytic activity test was conducted. The catalytic activity test was carried out, while the sample was heated at 750°C. The compositions of the gas fed to the apparatus employed in the catalytic activity test and the gas exhausted from the apparatus were analyzed by means of a microgas chromatograph (3000A, product of Agilent). The $C_2$ yield at 750°C was calculated on the basis of the compositional analysis ("YC$_2$-750," in FIGs. 5 and 6). Notably, $C_2$ HYDRO-CARBON contained in the gas exhausted from the above apparatus included ethylene and ethane. However, the ethylene content of the obtained $C_2$ HYDROCARBON was about 60 to about 70% in all samples (no data shown).

As shown in FIGs. 5 and 6, samples A1 to A29 provided a $C_2$ yield higher than that of samples A30 to A34. Thus, when the perovskite-type oxide forming the primary phase of the dehydrogenation reaction catalyst satisfied the aforementioned formula (1) and (3a) to (3d), the catalytic activity intrinsic to the dehydrogenation reaction catalyst was found to be enhanced. More specifically, when the element A' (at least one of lanthanum (La) and yttrium (Y)) is present in the A sites of the perovskite structure, "x≤0.4" ((3a)) or "x≤0.2" ((3e)) is conceived to be preferred, on the basis of comparison of, for example, sample A13 with sample A32. In addition, it has been confirmed that, particularly when "x=0" was achieved, the catalytic activity was prone to be enhanced, on the basis of comparison of, for example, sample A5 with samples A13 and A14, and of sample A21 with sample A25. Further, it has been confirmed that, when the element A, an alkaline earth metal in formula (1) was barium (Ba), the catalytic activity was prone to be enhanced, on the basis of comparison of, for example, sample A5 with samples A18 and A21; of sample A1 with samples A20 and A23; and of sample A6 with samples A19 and A22. In conclusion, it has been confirmed that the primary phase of the dehydrogenation reaction catalyst preferably has a perovskite structure represented by the aforementioned general formula (4).

Also, "$0 \leq z \leq 0.7$" is conceived to be preferred, on the basis of comparison of, for example, samples A1 to A5 with sample A30, and of samples A5 to A7 with sample A31. In addition, it has been confirmed that, when "$0<z$" was achieved (particularly when "$0.2 \leq z$" was achieved), the catalytic activity was prone to be enhanced, on the basis of comparison of, for example, samples A1 to A4 with sample A5, and of samples A6 and A7 with sample A5.

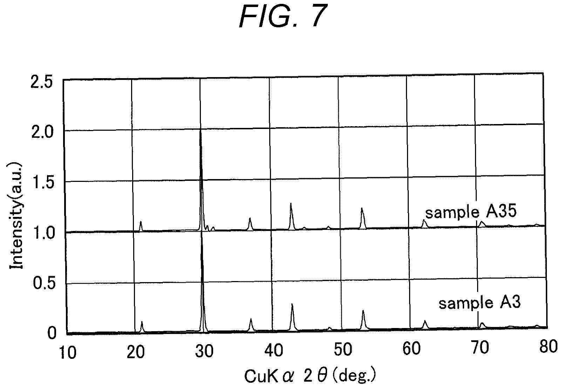

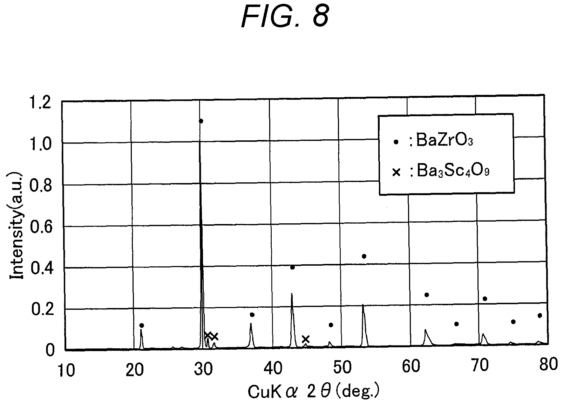

FIG. 7 is an XRD chart of dehydrogenation reaction catalysts of the first embodiment. FIG. 8 is an XRD chart of dehydrogenation reaction catalysts of the first embodiment with some descriptions. More specifically, FIG. 7 is an XRD chart of samples A3 and A35. FIG. 8 is an XRD chart of samples A35 shown in FIG. 7, with some descriptions. Both samples A3 and A35 are represented by the same compositional formula ($BaZr_{0.4}Sc_{0.6}O_3$), but the production methods therefor are different from each other. In the present disclosure, variation in methane oxidative coupling catalyst performance by the presence of the secondary phase in the dehydrogenation reaction catalyst was investigated.

[Production of sample A35]

Sample A35 ($BaZr_{0.4}Sc_{0.6}O_3$) was prepared through a solid phase reaction method. As raw material powders, barium carbonate (product of Sakai Chemical Industry Co., Ltd.), zirconium oxide (product of Daiichi Kigenso Kagaku Kogyo Co., Ltd.), and scandium oxide (product of Alfa Aesar) were used. These raw material powders were weighed so that the proportion of metal elements were adjusted to establish the composition of a compositional formula $BaZr_{0.4}Sc_{0.6}O_3$. To the mixture, boulders (diameter: 5 mm) and ethanol were added, and the resultant mixture was sufficiently mixed by means of a ball mill for 15 hours. Thereafter, an ethanol component was evaporated, to thereby yield a solid. The solid was sieved through a sieve (opening: 250 $\mu$m) and, then fired at 1,300°C for 5 hours. To the thus-obtained powder, boulders (diameter: 5 mm) and ethanol were added, and pulverization was conducted again by means of a ball mill for 15 hours with mixing. Thereafter, an ethanol component was evaporated, to thereby yield a solid. The solid was sieved through a sieve (opening: 250 $\mu$m) and, then fired at 1,300°C (heat treatment temperature) for 5 hours. To the thus-obtained powder, boulders (diameter: 5 mm) and ethanol were added, and pulverization was conducted again by means of a ball mill for 15 hours with mixing. Thereafter, an ethanol component was evaporated, to thereby yield a solid. The solid was sieved through a sieve (opening: 250 $\mu$m), to thereby yield a dehydrogenation reaction catalyst as sample A35. Notably, as mentioned above, sample A3 was prepared through a complex polymerization method.

As shown in FIG. 7, a peak attributed to the secondary phase which was not observed in the XRD chart of sample A3 was observed in the XRD chart of sample A35. That is, sample A35 was found to have a secondary phase in addition to the primary phase forming the perovskite-type oxide of sample A3. Specifically, as shown in FIG. 8, in the XRD chart of sample A35, there have been shown the first peak attributed to the perovskite-type oxide ($BaZr_{0.4}Sc_{0.6}O_3$) forming the primary phase and having the highest intensity present within a diffraction angle $2\theta$ range of 29.5 to 30.5°, and the second peak attributed to the complex oxide ($Ba_3Sc_4O_9$) forming the secondary phase and having the highest intensity present within a diffraction angle $2\theta$ range of 30.6 to 31.0°. That is, in sample A35, a primary phase formed of a $BaZrO_3$-based material and a secondary phase formed of $Ba_3Sc_4O_9$ were found to be co-present. Notably, the numbers of the database employed in FIG. 8; i.e., ICDD (International Centre for Diffraction Data) are 00-001-0890 corresponding to the perovskite-type oxide, and 00-031-0161 corresponding to $Ba_3Sc_4O_9$. Also, there has been known that the peak attributed to $Ba(Zr_{1-x}Sc_x)O_{3-\delta}$ is observed at a position slightly shifted on the lower angle side from the peak attributed to $BaZrO_3$. Accordingly, the position of the peak attributed to $BaZrO_3$ in the XRD chart shown in FIG. 8 was regarded as a peak attributed to $BaZr_{0.4}Sc_{0.6}O_3$.

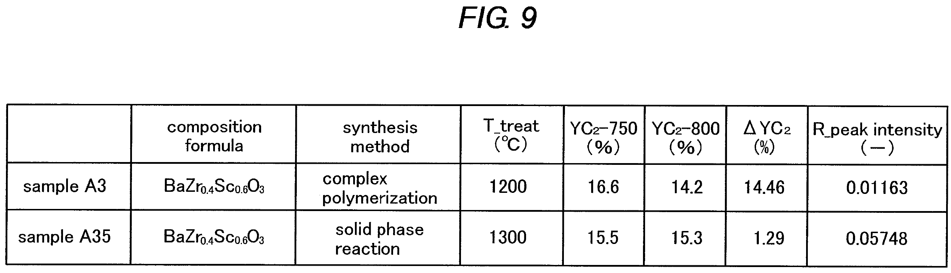

FIG. 9 is a first table showing a $C_2$ yield obtained by dehydrogenation reaction catalysts of the present embodiment. FIG. 9 shows $C_2$ yield values of samples A3 and A35, at various temperatures with peak intensity ratios indicated in the XRD chart of FIG. 7.

[Determination of $C_2$ yield]

The $C_2$ yields at 750°C ("$YC_2$-750," in FIG. 9) and 800°C ("$YC_2$-800," in FIG. 9) of samples A3 and A35 were measured by means of the aforementioned measurement apparatus. From the thus-determined $C_2$ yields at 750°C and 800°C, a percent drop in $C_2$ yield (800°C/750°C) ("$\Delta YC_2$," in FIG. 9) was calculated.

[Derivation of peak intensity ratio]

Samples A3 and A35 were subjected to a powder XRD analysis, to thereby determined a peak intensity ratio (secondary phase/primary phase). An X-ray diffractometer, SmartLab-3kw (product of Rigaku) was employed. The powder XRD measurement conditions included scanning range (2θ): 10° to 80°, step width: 0.02°, scanning speed: 20°/min, and optical system: Bragg-Brentano. As an X-ray source, CuKα ray was employed at a tube voltage of 40 kV and a tube current of 30 mA. The obtained powder X-ray diffraction pattern was subjected to a data analysis through the following procedure. Data smoothing was performed through weight-averaging of seven points. Background removal was performed through a Sonnevelt-Visser method at a peak width threshold of 0.1 and an intensity threshold of 0.01. Kα2 removal was performed at an intensity ratio of 0.5. In the powder X-ray diffraction pattern with a CuKα ray obtained through aforementioned procedure, a first peak, exhibiting the highest peak intensity attributed to a perovskite-type oxide forming the primary phase, and a second peak, exhibiting the highest peak intensity attributed to a complex oxide forming the secondary phase, were characterized. Thus, the ratio in intensity of the second peak to the first peak ("R_peak intensity," in FIG. 9) was determined.

As shown in FIG. 9, sample A35 was found to have a high $C_2$ yield at 800°C, as compared with sample A3, and a percent drop in $C_2$ yield (800°C to 750°C) was found to be very small. That is, when the secondary phase formed of a complex oxide generated in the perovskite-type oxide during production of the above perovskite-type oxide ($Ba_3Sc_4O_9$ in the case of sample A35) was added to the primary phase formed of a perovskite-type oxide having a catalytic activity intrinsic to a dehydrogenation reaction catalyst ($BaZr_{0.4}Sc_{0.6}O_3$ in the case of sample A35), the heat resistance intrinsic to a dehydrogenation reaction catalyst was found to be enhanced, and a drop in catalytic activity intrinsic to a dehydrogenation reaction catalyst was found to be suppressed under high temperature conditions (e.g., 800°C).

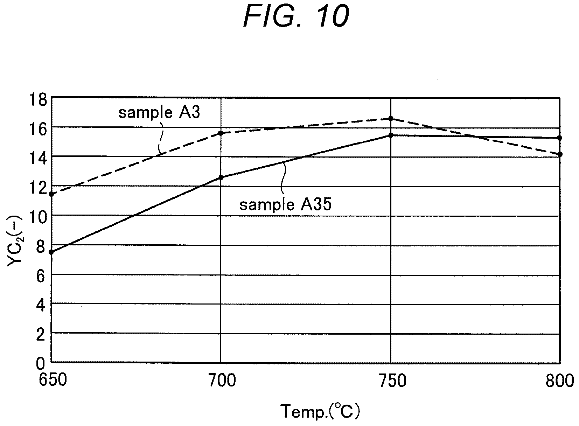

FIG. 10 is a table showing a variation in $C_2$ yield vs. temperature obtained by the dehydrogenation reaction catalysts of the present embodiment. FIG. 10 shows the results of $C_2$ yields of samples A3 and A35 measured after heating at 650°C, 700°C, 750°C, and 800°C, respectively. Among these results, the aforementioned FIG. 9 only shows the measurement results after heating at 750°C and 800°C.

The complex polymerization method, which is a sample A3 production method, allows raw materials to be mixed at a molecular level. Thus, as compared with the solid-phase reaction method, the complex polymerization method is generally known as a production method that can provide a catalyst having high catalytic activity. FIG. 10 shows that sample A3 exhibits a $C_2$ yield higher than that of sample A35, under temperature conditions of 700°C and 750°C. However, when the temperature of the dehydrogenation reaction catalyst in working rose to 800°C, sample A3 exhibited a drop in catalytic activity intrinsic to a dehydrogenation reaction catalyst ($C_2$ yield). In contrast, sample A35 prepared through the solid-phase reaction method and having a secondary phase exhibited a suppressed drop in catalytic activity even under high temperature conditions (e.g., 800°C). Thus, the dehydrogenation reaction catalyst; i.e., sample A35, prepared through the solid-phase reaction method was found to exhibit an enhanced heat resistance. Conceivably, the enhancement in heat resistance of sample A35 may be attributable to heat treatment temperature in preparation of a catalyst, and the presence of a secondary phase ($Ba_3Sc_4O_9$).

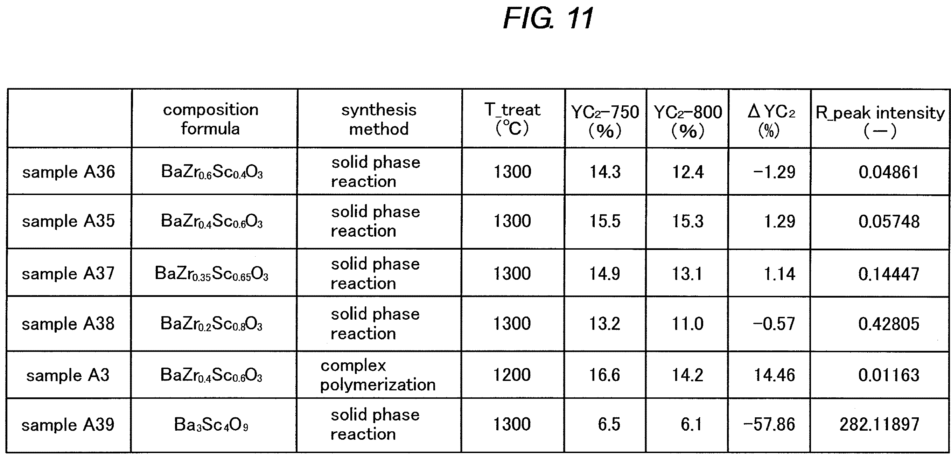

FIG. 11 is a second table showing a $C_2$ yield obtained by dehydrogenation reaction catalysts of the present embodiment. FIG. 11 shows the effect of the presence of a plurality of phases on catalytic performance. FIG. 11 shows characteristics of samples A36 to A38, prepared by modifying proportions of zirconium and scandium in the primary phase so as to differ from sample A35 having a primary phase of $BaZr_{0.4}Sc_{0.6}O_3$, and sample A39 ($Ba_3Sc_4O_9$) prepared through the solid-phase reaction method. Specifically, FIG. 11 shows $C_2$ yields at different temperatures ("YC_2-750" and "YC_2-800," in FIG. 11), a percent drop in $C_2$ yield (800°C/750°C) ("ΔYC_2," in FIG. 11), and a peak intensity ratio obtained from an XRD chart ("R_peak intensity," in FIG. 11). The $C_2$ yield and the peak intensity ratio obtained from an XRD chart shown in FIG. 11 were determined through the same methods as described in FIG. 9. Notably, FIG. 11 also shows the evaluation results of sample A3 shown in FIG. 9.

[Production of samples A36 to A39]

Samples A36 to A39 were prepared through a method in accordance with the aforementioned production method for sample A35. Specifically, the powder-form dehydrogenation reaction catalysts of samples A36 to A39 were yielded in a manner similar to that for producing sample A35, except that the type and weighing amount of each catalyst raw material were tuned to attain the compositional proportions of samples A36 to A39 having the respective compositional formula shown in FIG. 11, and the temperature of heat treatment the catalyst powder precursor ("T-treat") was modified to a temperature shown in FIG. 11. As raw material powders, barium carbonate (product of Sakai Chemical Industry Co., Ltd.), zirconium oxide (product of Daiichi Kigenso Kagaku Kogyo Co., Ltd.), and scandium oxide (product of Alfa Aesar) were used. Notably, samples A36 to A39 include a sample which had been received the heat treatment at a frequency different from that for producing sample A35. However, the number of times of heat treatment may vary.

As shown in FIG. 11, as compared with the case of sample A3, the temperature of the heat treatment of samples A35 to A38 during catalyst preparation was higher. In addition, formation of a secondary phase was observed in samples A35 to A38. According to the peak intensity (R_peak intensity) in XRD, the secondary phase was found to be present at a ratio of 0.04 or more with respect to the primary phase. As shown in relation to sample A39, $Ba_3Sc_4O_9$ serving as the secondary phase exhibited no deterioration in $C_2$ yield due to a rise in temperature. In contrast, the catalytic performance of sample A39 was found to be lower than that of a Ba(Zr,Sc)$O_3$-type material. In samples A35 to A38, a percent drop of $C_2$ yield (Y$C_2$-800) at 800°C to $C_2$ yield (Y$C_2$750) at 750°C ("ΔY$C_2$") was found to fall within a range of ±1.3%. Therefore, samples A35 to A38 were found to maintain relatively high catalytic activity and exhibite enhanced heat resistance.

<Example 2>

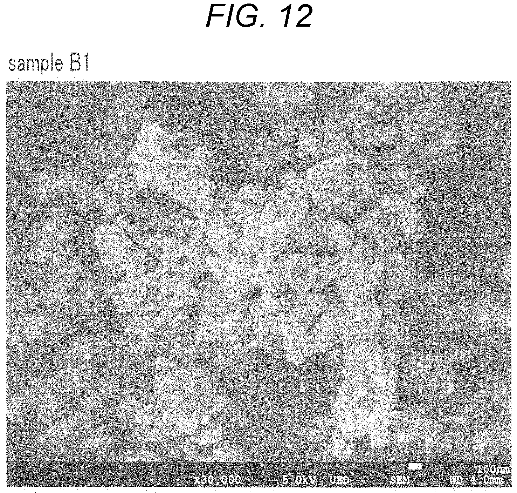

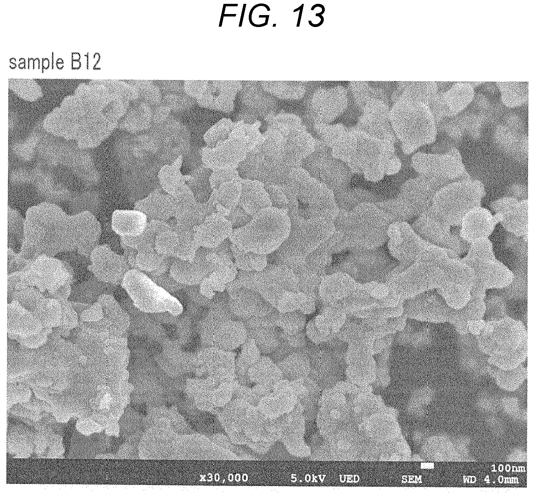

FIG. 12 is a first SEM image of the dehydrogenation reaction catalyst of the present embodiment. FIG. 13 is a second SEM image of the dehydrogenation reaction catalyst of the present embodiment. FIG. 12 is an SEM image of sample B1 produced through a solid phase reaction method, and FIG. 13 is an SEM image of sample B12 produced through a complex polymerization method. In Example 2, the samples of the dehydrogenation reaction catalyst produced through two different production methods were evaluated in terms of surface morphology varied by the difference in production method.

[Production of sample B1]

Sample B1 (BaZr$_{0.8}$Y$_{0.2}$O$_3$) was prepared through a complex polymerization method in accordance with a production method for sample A35. As raw material powders, barium carbonate (product of Sakai Chemical Industry Co., Ltd.), zirconium oxide (product of Daiichi Kigenso Kagaku Kogyo Co., Ltd.), and yttrium oxide (product of Shin-Etsu Chemical Co., Ltd.) were used. These raw material powders were weighed so that the proportion of metal elements were adjusted to establish the composition of a compositional formula BaZr$_{0.8}$Y$_{0.2}$O$_3$. To the mixture, boulders (diameter: 5 mm) and ethanol were added, and the resultant mixture was sufficiently mixed by means of a ball mill for 15 hours. Thereafter, an ethanol component was evaporated, to thereby yield a solid. The solid was sieved through a sieve (opening: 250 μm) and, then fired at 1,300°C for 5 hours (firing step). To the thus-obtained powder, boulders (diameter: 5 mm) and ethanol were added, and pulverization was conducted again by means of a ball mill with for 15 hours with mixing (pulverization step). Thereafter, an ethanol component was evaporated, to thereby yield a solid. The solid was sieved through a sieve (opening: 250 μm), to thereby yield a dehydrogenation reaction catalyst as sample B1.

[Production of sample B12]

Sample B12 (BaZr$_{0.8}$Y$_{0.2}$O$_3$) was prepared through a solid phase reaction method in accordance with a production method for sample A1. As raw material powders, barium nitrate (product of FUJIFILM Wako Pure Chemical Corporation), zirconyl nitrate (product of FUJIFILM Wako Pure Chemical Corporation), and yttrium nitrate (product of Sigma Aldrich) were used. These raw material powders were weighed so that the proportion of metal elements were adjusted to establish the composition of a compositional formula BaZr$_{0.8}$Y$_{0.2}$O$_3$. To the mixture of the above raw material powders, aqueous citric acid and propylene glycol were added so that the proportions: metal element : citric acid : propylene glycol were adjusted to 1:3:3 (by mole). The resultant mixture was agitated and sufficiently mixed at 80°C for 1 hour. The resultant solution was slowly heated to 300°C, to thereby yield a polymer in which the metals were dispersed. The thus-obtained polymer was heated at 400°C for 2 hours, and the formed carbonized product was pulverized by means of an agate mortar, to thereby yield a precursor of a catalyst powder. The thus-obtained catalyst powder precursor was subjected to a heat treatment at 1,200°C for 6 hours, to thereby yield a powder-form dehydrogenation reaction catalyst as sample B12.

When the SEM image of sample B1 shown in FIG. 12 was compared with the SEM image of sample B12 shown in FIG. 13, the surface morphology considerably differs from each other at a micron level, under the same composition (BaZr$_{0.8}$Y$_{0.2}$O$_3$). More specifically, sample B1 produced through a solid-phase reaction method was found to have a surface morphology more finer than that of sample B12 produced through a complex polymerization method. While the solid-phase reaction method for producing sample B1 included a pulverization step by means of a ball mill, the complex polymerization method for producing sample B12 included no pulverization step. Thus, as shown in FIGs. 12 and 13, differences in surface morphology and the size of grain masses determining the surface morphology were conceivably provided.

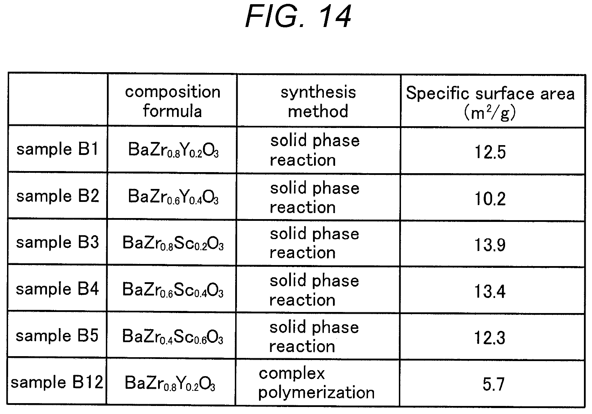

FIG. 14 is a graph showing specific surface areas of the dehydrogenation reaction catalysts of the present embodiment. FIG. 14 shows the effects of composition and production method in production of the dehydrogenation reaction catalyst on the specific surface area of the dehydrogenation reaction catalyst. FIG. 14 shows, in a comparative manner, measured specific surface areas of a plurality of samples B1 to B5 having different compositions and produced through a solid phase reaction method and sample 12 produced through a complex polymerization method.

[Production of samples B2 to B5]

Samples B2 to B5 were prepared through a method in accordance with the aforementioned production method for sample B1. More specifically, powder-form dehydrogenation reaction catalysts of samples B2 to B5 were produced in a manner similar to production of sample B1, except that the type and weighing amount of a catalyst raw material powder were changed to attain the compositional proportions of the compositional formulas of samples B2 to B5 shown in FIG. 14. Alternatively, samples B3 to B5 may be prepared by repeatedly conducting a firing step (heat treatment temperature: 1,300°C; firing time: 5 hours) and a pulverization step by means of a ball mill, to thereby yield corresponding powder-form dehydrogenation reaction catalysts. As raw material powders, barium carbonate (product of Sakai Chemical Industry Co., Ltd.), zirconium oxide (product of Daiichi Kigenso Kagaku Kogyo Co., Ltd.), yttrium oxide (product of Shin-Etsu Chemical Co., Ltd.), and scandium oxide (product of Alfa Aesar) were used.

[Measurement of specific surface area]

The specific surface area of each of samples B1 to B5 and sample B12 was measured through a BET flow single-point method (He:$N_2$=7:3) by means of a specific surface area meter ( Macsorb HM model-1208, product of Mountech Co., Ltd.). In a specific procedure, a particle sample of each of samples B1 to B5 and sample B12 was charged into a test tube, and the sample was degassed at 200°C for 60 minutes. The test tube containing the degassed sample was cooled. After cooling, the amount of gas physically adsorbed on the surfaces of the particles charged in the test tube was measured on the basis of a change in concentration, to thereby calculate a specific surface area of the particle sample ("Specific surface area," in FIG. 14).

As shown in FIG. 14, showing comparison of the specific surface area of sample B1 with the specific surface area of sample B12, the specific surface area of a dehydrogenation reaction catalyst was found to be varied in response to a change in surface morphology as shown in FIGs. 12 and 13. More specifically, the specific surface area of sample B1 was found to be twice or more the specific surface area of sample B12. As a result, reactivity of the sample with the dehydrogenation reaction catalyst (per unit weight) will conceivably rise. Samples B1 to B5 had a specific surface area of measured through a BET flow single-point method of 10 $m^2$/g or more. For example, on the basis of sample B1 with sample B3, the specific surface area was found to increase, when the B site element was scandium rather than yttrium. On the basis of comparison of sample B1 with sample B2 or among samples B3 to B6, the specific surface area was found to increase, when the zirconium content increased.

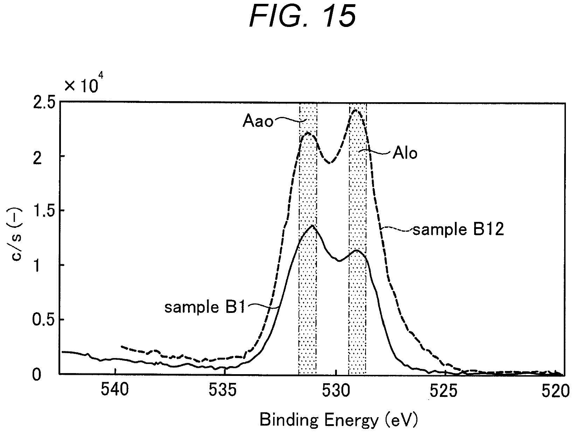

FIG. 15 is a first XPS spectrum of the dehydrogenation reaction catalysts of the present embodiment. FIG. 15 shows an O 1s orbital spectrum of each of samples B1 and B12 included in a photoelectron spectrum obtained through an X-ray photoelectron spectroscopy.

[Measurement of O 1s orbital spectrum]

An O 1s orbital spectrum of each of samples B1 and B12 was obtained through an X-ray photoelectron spectroscopy (XPS). In a specific procedure, particles of sample B1 or B12 was pressed to form a compact. A broken face of the compact was irradiated with a monochromatic AlK$\alpha$ ray as an X-ray source having a pass energy of 140 eV, to thereby obtain an XPS spectrum within a range of 100 $\mu m \varphi$.

In each of the O 1s orbital spectrum of sample B1 and that of sample B12 shown in FIG. 15, two peaks were observed. Among the two peaks in each O 1s orbital spectrum, the peak observed at a binding energy of about 532 eV (area Aao, in FIG. 15) is attributed to an adsorbed oxygen species, and the peak observed at a binding energy of about 528 eV (area Alo, in FIG. 15) is attributed to an in-lattice oxygen species. Regarding samples B1 and B12, respectively, the area of a convex part including a peak attributed to an adsorbed oxygen species was compared with the area of a convex part including a peak attributed to an in-lattice oxygen species. As a result, in sample B12, the area of a convex part including a peak attributed to an in-lattice oxygen species was found to be wider than the area of a convex part including a peak attributed to an adsorbed oxygen species. In contrast, in sample B1, the area of a convex part including a peak attributed to an adsorbed oxygen species was found to be wider than the area of a convex part including a peak attributed to an in-lattice oxygen species. In the case of sample B1, since the solid-phase reaction method of producing sample B1 includes a pulverization step by means of a ball mill, a surface suitable for adsorption of oxygen as shown in the SEM image of FIG. 12 is conceivably formed by mechanical force. In contrast, in the case of sample B12, since the complex polymerization method of producing sample B12 includes no step of applying mechanical force (e.g., a pulverization step as employed in the solid-phase reaction method), a surface having such surface morphology as observed in sample B1 was not observed, as shown in the SEM image of FIG. 13. Thus, since sample B1 and sample B12 differed from each other in surface morphology due to presence or absence of a pulverization step, the magnitude relation between the amount of adsorbed oxygen species and that of in-lattice oxygen species conceivably varied. Notably, the results of comparison of areas of a convex part including a peak in an XPS spectrum will be specifically described in the below-described section.

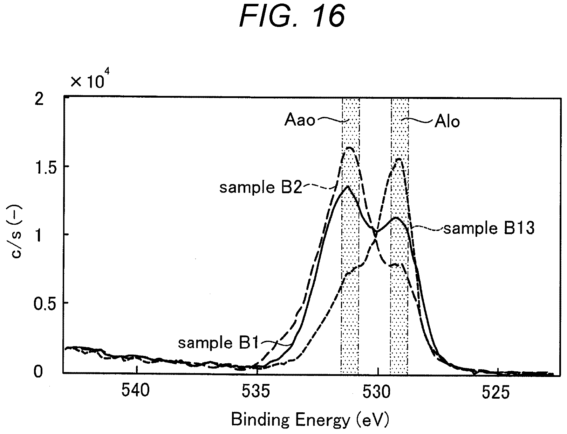

FIG. 16 is a second XPS spectrum of the dehydrogenation reaction catalysts of the present embodiment. FIG. 16 shows an O 1s orbital spectrum of a photoelectron spectrum obtained through X-ray photoelectron spectroscopy of each of samples B1, B2, and B13. All samples B1, B2, and B13 shown in FIG. 16 were dehydrogenation reaction catalysts having the same compositional formula of $Ba(Zr_{1-x}Y_x)O_{3-\delta}$, but the value of x was varied (sample B1: x=0.2, sample B2: x=0.4, and sample B13: x=0).

[Production of sample B13]

Sample B13 ($BaZrO_3$) was prepared through a solid phase reaction method in accordance with the aforementioned production method for sample B1. Specifically, the powder-form dehydrogenation reaction catalyst of samples B13 was yielded in a manner similar to that for producing sample B1, except that the type and weighing amount of each catalyst raw material were tuned to attain the compositional proportions of sample B13. As raw material powders, barium carbonate (product of Sakai Chemical Industry Co., Ltd.) and zirconium oxide (product of Daiichi Kigenso Kagaku Kogyo Co., Ltd.) were used.

As shown in FIG. 16, in the dehydrogenation reaction catalyst represented by the compositional formula $Ba(Zr_{1-x}Y_x)O_{3-\delta}$, the greater the value of x, the easier the achievement of an oxygen deficiency for compensating electric charge. In the three samples shown in FIG.16, sample B2 having an x greater than that of sample B1 was found to have a ratio of the area of a convex part including a peak attributed to an adsorbed oxygen species to the area of a convex part including a peak attributed to an in-lattice oxygen species, which was greater than that of sample B1. Also, sample B13 having an x smaller than that of sample B1 was found to have a ratio of the area of a convex part including a peak attributed to an adsorbed oxygen species to the area of a convex part including a peak attributed to an in-lattice oxygen species, of smaller than that of sample B1. That is, when the value of x increased, the relative area of a convex part including a peak attributed to an adsorbed oxygen species to the area of a convex part including a peak attributed to an in-lattice oxygen species was found to tend to increase. In the dehydrogenation reaction catalyst represented by the compositional formula $Ba(Zr_{1-x}Y_x)O_{3-\delta}$, it has been conceived that the oxygen deficiency increases as increase in the value of x, whereby the degree of coordination unsaturation at the catalyst surface near the oxygen deficient region increases. As a result, conceivably, a larger amount of oxygen can be adsorbed, whereby the amount of adsorbed oxygen species increases.

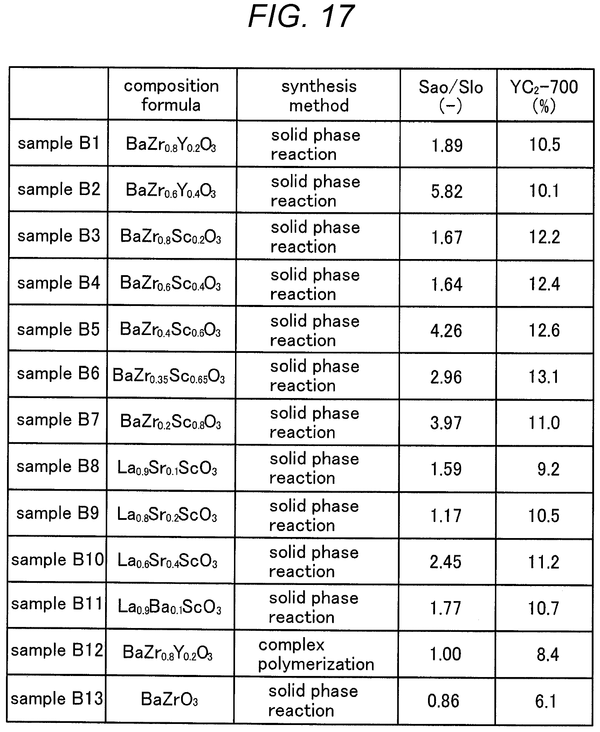

FIG. 17 is a third table showing a $C_2$ yield obtained by the dehydrogenation reaction catalysts of the present embodiment. FIG. 17 shows the relationship between the composition of the dehydrogenation reaction catalyst and $C_2$ yield. FIG. 17 shows the ratio of the peak area attributed to an adsorbed oxygen species to the peak area attributed to an in-lattice oxygen species in the O 1s orbital spectrum obtained through XPS measurement (hereinafter may be referred to simply as "XPS ratio"), with respect to samples B1 to B11 and B13 prepared through the solid-phase reaction method, and sample B12 prepared through the complex polymerization method, along with $C_2$ yield at 700°C.

[Production of samples B6 to B11]

Samples B6 to B11 were prepared through a method in accordance with the aforementioned production method for sample B1. More specifically, powder-form dehydrogenation reaction catalysts of samples B6 to B11 were produced in a manner similar to production of sample B1, except that the type and weighing amount of a catalyst raw material powder were changed to attain the compositional proportions of the compositional formulas of samples B6 to B11 shown in FIG. 17. Alternatively, similar to sample B5, samples B6 and B7 may be prepared by repeatedly conducting a firing step and a pulverization step by means of a ball mill, to thereby yield corresponding powder-form dehydrogenation reaction catalysts. In the cases of samples B8 to B11, the heat treatment temperature in the firing step was modified to fall within a range of 1,300°C to 1,350°C, so as to attain high target substance content. For example, the temperature was 1,400°C in the case of sample B10, and the temperature was 1,350°C in the case of sample B11. Thus, the heat treatment temperature during the firing step in production of the samples may be modified so as to attain high target substance content. As raw material powders, barium carbonate (product of Sakai Chemical Industry Co., Ltd.), zirconium oxide (product of Daiichi Kigenso Kagaku Kogyo Co., Ltd.), scandium oxide (product of Alfa Aesar), lanthanum hydroxide (product of Shin-Etsu Chemical Co., Ltd.), and strontium carbonate (product of Kojundo Chemical Laboratory Co., Ltd.) were used.

[Determination of XPS ratio]

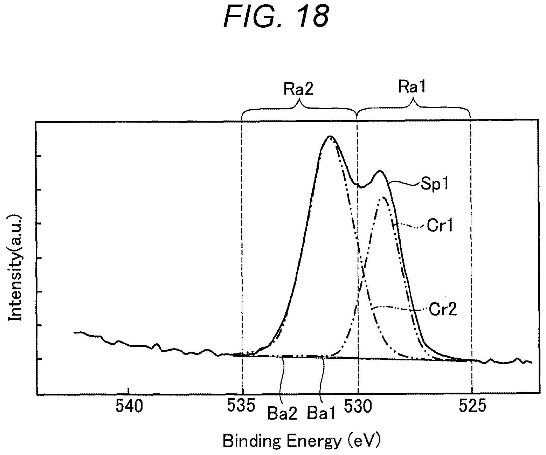

FIG. 18 is a graph showing calculation of XPS ratios. In calculation of an XPS ratio, as shown in FIG. 18, an O1s orbital spectrum Sp1 obtained through XPS is split, through a peak separation fitting processing, to a first curve Cr1 including a first peak attributed to a maximum value of binding energy within a range Ra1 of 525 to 530 eV, and a second curve Cr2 including a second peak attributed to a maximum value of binding energy within a range Ra2 of 530 to 535 eV. A base line Ba1 serving as a base of the first curve Cr1 is drawn, and the area of the region enclosed by the first curve Cr1 and the base line Ba1 is defined as a first peak area Slo attributed to an in-lattice oxygen species. A base line Ba2 serving as a base of the second curve Cr2 is drawn, and the area of the region enclosed by the second curve Cr2 and the base line Ba2 is defined as a second peak area Sao attributed to an adsorbed oxygen species. Thus, the XPS ratio is derived as Sao/Slo. In FIG. 17, the XPS ratio is denoted by "Sao/Slo." The first peak area Slo corresponds to the "area of a convex part including the first peak" in the Claims, and second peak area Sao corresponds to the "area of a convex part including the second peak" in the Claims.

[Determination of $C_2$ yield]

The $C_2$ yield obtained by samples B1 to B13 was determined by means of an immobilized bed flow reactor, similar to the aforementioned determination of $C_2$ yield obtained by samples A1 to A34. In a specific procedure, each of samples B1 to B13 was weighed to take a portion of 0.1 g and placed in an immobilized bed flow reactor. While the reactor was heated at a predetermined temperature, a gas mixture containing methane, oxygen, and nitrogen was caused to flow through the reactor at a pressure of 1 atm, a flow rate of 45 $cm^3$/min, and flow proportions $CH_4$:$O_2$:$N_2$ of 3.8:1:4. Under the conditions, a catalytic activity test was conducted. The "$YC_2$-700" shown in FIG. 17 is a yield obtained each sample heated at 700°C.

As shown in FIG. 17, when the XPs ratio ("Sao/Slo," shown in FIG. 17) increased, the $C_2$ yield at 700°C ("$YC_2$-700," shown in FIG. 17) was found to tend to increase. Particularly, samples B1 to B11, exhibiting an XPS ratio; i.e., the ratio of second peak area to first peak area, of greater than 1, the $C_2$ yield at 700°C was found to tend to further increase. In the dehydrogenation reaction catalyst, the adsorbed oxygen species is present in an activated state, whereby dehydrogenation reactivity of the dehydrogenation reaction catalyst is enhanced. High XPS ratio, a ratio of the first peak area attributed to an adsorbed oxygen species to the second peak area attributed to an in-lattice oxygen species means presence of a relatively large amount of adsorbed oxygen species. Thus, when the XPS ratio is high, dehydrogenation reactivity of the dehydrogenation reaction catalyst is enhanced.

As shown in FIG. 17, in order to enhance the XPS ratio of the dehydrogenation reaction catalyst, conceivably, there must be satisfied the following conditions (a) and (b): (a) "the dehydrogenation reaction catalyst production method includes a pulverization step employed in the solid-phase reaction method." and (b) "oxygen deficiency is provided through element substitution." Through a synergistic effect of the two conditions (a) and (b), the XPS ratio of the dehydrogenation reaction catalyst can be enhanced. Among the plurality of samples shown in FIG. 17, samples B1 to B11 satisfy both the two conditions (a) and (b). In contrast, sample B12 satisfies the condition (b), but does not satisfy the condition (a). Sample B13 satisfies the condition (a), but does not satisfy the condition (b).

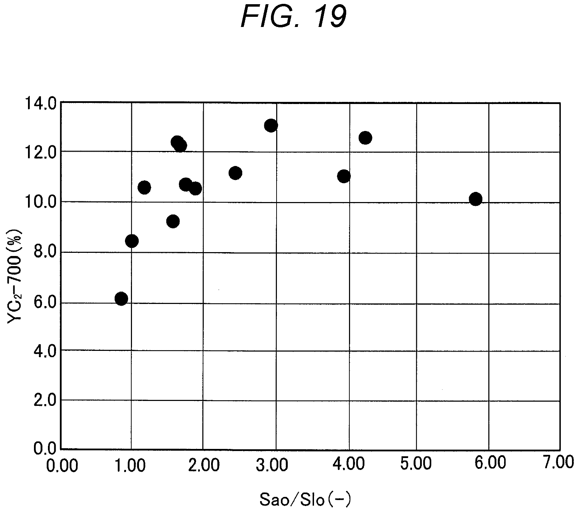

FIG. 19 is a fourth table showing $C_2$ yields obtained by dehydrogenation reaction catalysts of the present embodiment. FIG. 19 shows the relationship between the calculated XPS ratios and $C_2$ yields. FIG. 19 is a graph having a horizontal axis representing XPS ratio (Sao/Slo), and a vertical axis representing $C_2$ yield at 700°C ($YC_2$-700), in which numerical data of the 13 samples B1 to B13 shown in FIG. 17 were plotted. As shown in FIG. 19, when the XPS ratio was greater than 1, $C_2$ yield at 700°C was found to be enhanced. Conceivably, the enhancement is achieved, a substantial increase in activated adsorbed oxygen species at the surface of the dehydrogenation reaction catalyst, to thereby promote dehydrogenation reaction.

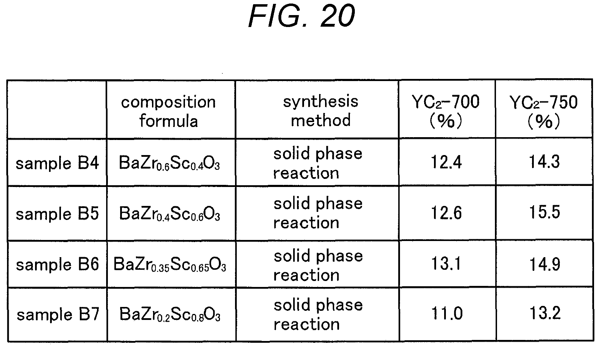

FIG. 20 is a fifth table showing $C_2$ yields obtained by the dehydrogenation reaction catalysts of the present embodiment. FIG. 20 shows temperature dependence in $C_2$ yield obtained by the dehydrogenation reaction catalyst. FIG. 20 shows $C_2$ yields at 750°C ($YC_2$-750) obtained by samples B4 to B7 among samples B1 to B13 shown in FIG. 17. Samples B4 to B7 are so-called Ba(Zr,Sc)$O_3$-based materials.

[Determination of $C_2$ yield]

The $C_2$ yield at 750°C obtained by samples B4 to B7 was determined by means of an immobilized bed flow reactor, similar to the aforementioned determination of $C_2$ yield to 700°C obtained by samples B1 to B13. The "$YC_2$-750" shown in FIG. 20 is a yield obtained by each sample heated at 750°C.

As shown in FIG. 20, all samples B4 to B7 were found to provide a $C_2$ yield at 750°C higher than a $C_2$ yield at 700°C. Therefore, in a dehydrogenation reaction catalyst formed of a perovskite-type oxide of a Ba(Zr,Sc)$O_3$-based material and produced through a solid-phase reaction method, no deterioration was found in performance intrinsic to dehydrogenation reaction catalyst due to a rise in temperature from 700°C to 750C°.

The dehydrogenation reaction catalyst of the present embodiment having the aforementioned characteristic features includes a primary phase formed of a perovskite-type oxide represented by a general formula $(A_{1-x}A'_x)(Zr_{1-y-z}B_yB'_z)O_{3-\delta}$ and a secondary phase formed of at least one member of three complex oxides represented by general formulas $AB'_2O_4$, $A_2B'_2O_5$, and $A_3B'_4O_9$, respectively. In the primary phase, A represents at least one element selected from alkaline earth metals; A' represents at least one element of lanthanum (La) and yttrium (Y); B represents at least one element of titanium (Ti) and cerium (Ce); B' represents at least one element selected from among yttrium (Y), scandium (Sc), ytterbium (Yb), aluminum (Al), indium (In), and neodymium (Nd); relationships: $0 \leq x \leq 0.4$, $0.3 \leq (1-z) \leq 1$, $0 \leq y$, and $0 < (1-y-z)$ are satisfied; and $\delta$ represents an oxygen deficiency. In the secondary phase, A and B' are the same elements as A and B' forming the perovskite-type oxide. Thus, the heat resistance of the dehydrogenation reaction catalyst can be enhanced.

In the dehydrogenation reaction catalyst of the present embodiment, A is barium (Ba), B' is scandium (Sc), and the secondary phase is formed of a barium scandate which is at least one member of three complex oxides: $BaSc_2O_4$, $Ba_2Sc_2O_5$, and $Ba_3Sc_4O_9$. Thus, the heat resistance intrinsic to a dehydrogenation reaction catalyst can be further enhanced.

In the dehydrogenation reaction catalyst of the present embodiment, the secondary phase is formed of $Ba_3Sc_4O_9$. Thus, the heat resistance intrinsic to a dehydrogenation reaction catalyst can be further enhanced.

In the dehydrogenation reaction catalyst of the present embodiment, the primary phase is formed of $BaZr_{1-z}Sc_zO_{3-\delta}$ (wherein a relationship $0.1 \leq z \leq 0.7$ is satisfied). Thus, the catalytic activity intrinsic to a dehydrogenation reaction catalyst can be further enhanced.

In the dehydrogenation reaction catalyst of the present embodiment, in a powder X-ray diffraction pattern obtained by a CuK$\alpha$ ray, the ratio in intensity of the second peak to the first peak is 0.04 or greater. Thus, the heat resistance intrinsic to a dehydrogenation reaction catalyst can be further enhanced.

In the dehydrogenation reaction catalyst of the present embodiment, the secondary phase is formed of at least one member of three complex oxides: $BaSc_2O_4$, $Ba_2Sc_2O_5$, and $Ba_3Sc_4O_9$. Thus, the heat resistance intrinsic to a dehydrogenation reaction catalyst can be further enhanced.

The dehydrogenation reaction catalyst of the present embodiment may be used as a methane oxidative coupling catalyst for producing a $C \geq 2$ hydrocarbon from methane. Thus, the heat resistance of a methane oxidative coupling catalyst can be enhanced.

In the dehydrogenation reaction catalyst of the present embodiment, in an O1s orbital spectrum included in a photoelectron spectrum obtained through X-ray photoelectron spectroscopy, the ratio of a second peak area to a first peak area is greater than 1. Thus, adsorption of an adsorbed oxygen species on the surface of the dehydrogenation reaction catalyst is facilitated, whereby the catalytic activity intrinsic to a dehydrogenation reaction catalyst can be enhanced.

The dehydrogenation reaction catalyst of the present embodiment has oxygen deficiency. Thus, the amount of adsorbed oxygen species present on the surface of the dehydrogenation reaction catalyst can be increased, whereby the catalytic activity intrinsic to a dehydrogenation reaction catalyst can be further enhanced.

In the dehydrogenation reaction catalyst of the present embodiment, the primary phase included in the catalyst has a perovskite structure. Thus, the catalytic activity intrinsic to a dehydrogenation reaction catalyst can be further enhanced.

In the dehydrogenation reaction catalyst of the present embodiment, the specific surface area as determined through the BET technique is $10 \, m^2/g$ or more. Thus, the amount of adsorbed oxygen species present on the surface of the dehydrogenation reaction catalyst is increased, whereby the catalytic activity intrinsic to a dehydrogenation reaction catalyst can be further enhanced.

The dehydrogenation reaction catalyst of the present embodiment may be used as a methane oxidative coupling catalyst for producing a $C \geq 2$ hydrocarbon from methane. Thus, the catalytic performance of a methane oxidative coupling catalyst can be enhanced.

<Second embodiment>

The dehydrogenation reaction catalyst of the second embodiment differs from the dehydrogenation reaction catalyst of the first embodiment in terms of compositional formula.

[Dehydrogenation reaction catalyst]

The dehydrogenation reaction catalyst of the present embodiment is a complex oxide having a perovskite-type oxide crystal structure represented by the below-mentioned general formula (26).

$$(La_{1-x}M1_x)M2O_{3-\delta} \cdots \qquad (26)$$

In the above formula (26), M1 represents at least one element selected from alkaline earth metals; M2 represents at least one element selected from among yttrium (Y), scandium (Sc), ytterbium (Yb), aluminum (Al), indium (In), and gallium (Ga); and $\delta$ represents an oxygen deficiency satisfying $0 \leq \delta \leq 0.6$.

In the dehydrogenation reaction catalyst of the present embodiment, the perovskite-type oxide of formula (26) has a catalytic activity intrinsic to dehydrogenation reaction catalyst. The perovskite-type oxide of formula (26) contains at least one element M1 selected from lanthanum (La) and an alkaline earth metal in so-called A sites of the perovskite structure. The element M1 is preferably selected from strontium (Sr) and barium (Ba). Also, perovskite-type oxide of formula (26) contains element M2 in so-called B sites of the perovskite structure. The element M2 includes at least one element selected from yttrium (Y), scandium (Sc), ytterbium (Yb), aluminum (Al), indium (In), and gallium (Ga).

In the perovskite-type oxide of formula (26), x satisfies the following formula (27). Thus, the dehydrogenation reaction catalyst of the present embodiment exhibits a considerably high catalytic activity intrinsic to dehydrogenation reaction catalyst; i.e., an activity for promoting dehydrogenation reaction.

$$0 \leq x \leq 0.6 \quad \cdots \quad (27)$$

The thus-provided dehydrogenation reaction catalyst of the present embodiment has a perovskite-type oxide crystal structure represented by formula (26). Thus, there can be enhanced the catalytic activity intrinsic to dehydrogenation reaction catalyst; i.e., an activity for promoting dehydrogenation reaction, at a relatively low temperature of, for example about 650°C to about 700°C.

The dehydrogenation reaction catalyst of the present embodiment can promote various dehydrogenation reactions for releasing hydrogen from a compound. Examples of the dehydrogenation reaction include a methane oxidative coupling (i.e., oxidative coupling of methane: OCM) reaction for producing a $C \geq 2$ hydrocarbon from methane. Thus, the dehydrogenation reaction catalyst of the present embodiment may also be employed as a methane oxidative coupling catalyst. Examples of the dehydrogenation reaction also include various dehydrogenation reactions for forming hydrogen from hydrocarbon-based compounds including hydrocarbon and alcohol. Specific examples thereof include steam reformation reaction (i.e., forming hydrogen from the aforementioned hydrocarbon-based compound and steam), partial oxidation reaction (i.e., forming hydrogen from the aforementioned hydrocarbon-based compound), and shift reaction (i.e., forming carbon dioxide and hydrogen from carbon monoxide and steam generated in the aforementioned partial oxidation reaction).

FIGs. 2 and 3 are schemes each showing an example of methane oxidative coupling reaction proceeding on a methane oxidative coupling catalyst, in the case where the dehydrogenation reaction catalyst of the present embodiment corresponds to the methane oxidative coupling. In proceeding of a dehydrogenation reaction for releasing hydrogen from a compound, a covalent bond between a hydrogen atom and another atom (e.g., a carbon atom forming the compound) is generally broken. The dehydrogenation reaction catalyst of the present embodiment exhibits high activity of promoting dehydrogenation reaction, conceivably because the catalyst promotes the reactions of the aforementioned modes. In methane oxidative coupling reaction, conceivably, a reaction for generating methyl radicals from methane (see the following formula (5)), and reactions (see the following formulas (6) and (7) in FIG. 2) or reactions (see the following formulas (8) and (9) in FIG. 3) proceed, to thereby form $C_2$ hydrocarbons such as ethylene. The overall reaction for forming ethylene via methane oxidative coupling reaction is represented by the following reaction (10).

$$CH_4 \rightarrow \cdot CH_3 + H^+ + e^- \; ... \quad (5)$$

$$2 \cdot CH_3 + 1/2 O_2 \rightarrow C_2H_4 + H_2O \; \cdots \quad (6)$$

$$1/2 O_2 + 2H^+ + 2e^- \rightarrow H_2O \; \cdots \quad (7)$$

$$2 \cdot CH_3 \rightarrow C_2H_6 \rightarrow C_2H_4 + 2H^+ + 2e^- \; \cdots \quad (8)$$

$$O_2 + 4H^+ + 4e^- \rightarrow 2H_2O \; \cdots \quad (9)$$

$$2CH_4 + O_2 \rightarrow C_2H_4 + 2H_2O \; \cdots \quad (10)$$

In the above case, the covalent bond between a carbon atom and a hydrogen atom of methane is considerably stable with a binding energy of 104 kcal/mol. Thus, conceivably, a reaction of generating methyl radicals through dehydrogenation of methane having the aforementioned stable covalent bond generally plays a role of a rate-determining step (i.e., reaction represented by formula (5)) in methane oxidative coupling reaction. Since the dehydrogenation reaction catalyst of the present embodiment exhibits a high activity of promoting a reaction involving cut of a covalent bond with a hydrogen atom (e.g., the aforementioned reaction of forming methyl radicals from methane), a wide range of dehydrogenation reactions involving cut of a covalent bond with a hydrogen atom (e.g., methane oxidative coupling reaction) can be conceivably promoted. Furthermore, the dehydrogenation reaction catalyst of the present embodiment is a perovskite-type oxide, serving as a proton conductor, whereby a hydrogen in a hydrogen-containing molecule is protonated and transferred. Thus, conceivably, the dehydrogenation reaction catalyst exhibits a high activity of promoting a reaction involving cut of a covalent bond with a hydrogen atom, whereby a wide range of dehydrogenation reactions involving cut of a covalent bond with a hydrogen atom (e.g., methane oxidative coupling reaction) can be promoted.

In FIGs. 2 and 3, a site where a reaction of formula (5) for forming methyl radicals from methane proceeds is denoted as an "oxidation site." FIGs. 2 and 3 show proceeding of a reaction of formula (6) or (8) for forming ethylene from methyl radical at the oxidation site. Also, in FIGs. 2 and 3, a site where a reaction of formula (7) or (9) for forming water by use of protons and electrons generated with methyl radicals according to formula (5) proceeds is denoted as a "reduction site." Thus, in proceeding of a reaction involving the dehydrogenation reaction in the dehydrogenation reaction catalyst of the present embodiment, transfer of protons and electrons generally is involved. Therefore, in order to enhance catalytic activity, the dehydrogenation reaction catalyst preferably has high activity of promoting the aforementioned reaction of formula (5), which is rate-determining step, and high proton conductivity and electron conductivity (including hole conductivity). Specifically, proton conductivity and proton transport number, or electron conductivity (including hole conductivity) and electron transport number (including hole transport number) are preferably higher. In addition, the entirety of the dehydrogenation reaction catalyst preferably has a higher whole conductivity, which is a sum of proton conductivity and electron conductivity (including hole conductivity).

[Other dehydrogenation reactions]

FIG. 4 is a representation of various examples of dehydrogenation reaction which the dehydrogenation reaction catalyst of the present embodiment can promote. The dehydrogenation reaction catalyst of the present embodiment can promote various reactions depending on the raw materials (reactants) employed. The formulas (11) to (22) in FIG. 4 each represent a reaction in which reactants including at least one of methane and a methane derivative (hereinafter may also be collectively referred to as "METHANE") are bound. Such reaction may also be called "METHANE oxidative coupling reaction." Also, the dehydrogenation reaction catalyst of the present embodiment for promoting METHANE oxidative coupling reaction may also be referred to as a "METHANE oxidative coupling catalyst." In formulas (13) to (22) in FIG. 4, each of the references A and B present in a methane derivative refers to an atom or a group serving as a substituent for hydrogen of a methane molecule, and n is an integer of 2 or greater. In each of the methane derivatives shown in FIG. 4,, one or two hydrogen atoms of the methane molecule are substituted by any of the aforementioned substituents. In the case where one molecule has substituents A and B, A and B may be the substituents identical to or different from each other. Examples of the substituent A or B include a halogen element, a hydroxy group (-OH), and a phenyl group ($-C_6H_5$).
In FIG. 4, similar to formula (10), formula (11) represents a reaction for forming ethylene from methane. Formula (12) represents a reaction involving a further polymerization reaction to form polyethylene. Differing from formula (10), formulas (11) to (22) represent only a change in methane and a methane derivative among the molecules involved in the relevant reaction.
Formulas (11) and (12) represent a reaction involving methane as a reactant. Formulas (13) and (14) represent a reaction involving a methane derivative represented by $BACH_2$ as a reactant. Formulas (15) and (16) represent a reaction involving a methane derivative represented by $ACH_3$ as a reactant. Formulas (17) and (18) represent a reaction involving a methane derivative represented by $ACH_3$ and methane as reactants. Formulas (19) and (20) represent a reaction involving a methane derivative represented by $ACH_3$ and a methane derivative represented by $CA_2H_2$ as reactants. Formulas (21) and (22) represent a reaction involving a methane derivative represented by $CABH_2$ and methane as reactants.
Formulas (11), (13), (15), (17), (19), and (21) each represent a reaction for forming a C2 hydrocarbon (ethylene) or an ethylene derivative (hereinafter may also be collectively referred to as a "$C_2$ HYDROCARBON") by use of a reactant selected from a C1 hydrocarbon (methane) and a methane derivative (hereinafter may also be collectively referred to as a "$C_1$ HYDROCARBON"). Formulas (12), (14), (16), (18), (20), and (22) each represent a reaction for forming a polymer by use of a reactant selected from the $C_1$ HYDROCARBONs. In formation of a polymer, after formation of a $C_2$ HYDRO-CARBON from a $C_1$ HYDROCARBON, $C_2$ HYDROCARBON molecules may be further polymerized. Alternatively, a $C_1$ HYDROCARBON may be successively polymerized at an end of a molecule, to thereby form a polymer. These polymerization reactions may proceed in parallel.
The reactions shown in FIG. 4 will be described in more detail. In formula (14), when each of the substituents A and B is a fluorine atom (F), polytetrafluoroethylene (PTFE) is yielded as a product. In formula (18), when the substituent A is a chlorine atom (Cl), poly(vinyl chloride) (PVC) is yielded as a product. When the substituent A is a hydroxy group (-OH), poly(vinyl alcohol) (PVOH) is yielded as a product. When the substituent A is a phenyl group ($-C_6H_5$), polystyrene (PS) is yielded as a product. In formula (22), when each of the substituents A and B is a fluorine atom (F), poly(vinylidene fluoride) (PVDF) is yielded as a product. When each of the substituents A and B is a chlorine atom (Cl), poly(vinylidene chloride) (PVDC) is yielded as a product. In formula (22), when the substituent A is a methyl group ($-CH_3$), and the substituent B is a methoxycarbonyl group ($-COOCH_3$), poly(methyl methacrylate) (PMMA) is yielded as a product.
In FIG. 4, specific METHANE oxidative coupling reactions are shown. However, the dehydrogenation reaction catalyst of the present embodiment may be used for promoting a dehydrogenation reaction other than the METHANE oxidative coupling reactions. Examples of the dehydrogenation reaction which the dehydrogenation reaction catalyst of the present embodiment can promote include a reaction in which reactants including at least one of an alkane and an alkane derivative (hereinafter may also be collectively referred to as "ALKANE") are bound. Such a reaction may also be referred to an "ALKANE oxidative coupling reaction" collectively with the aforementioned METHANE oxidative coupling reaction. The dehydrogenation reaction catalyst of the present embodiment which catalyst promotes the ALKANE oxidative coupling reaction may also be referred to as an "ALKANE oxidative coupling catalyst." The number of carbon atoms of the ALKANE serving as a reactant of the ALKANE oxidative coupling reaction which the dehydrogenation reaction catalyst of the present embodiment promotes may be, for example, 1 to 10, and is preferably 1 to 5, more preferably 1 or 2. Notably, the alkane derivative which is subjected to the ALKANE oxidative coupling reaction is derived by substituting hydrogen present in an alkane molecule with any of the aforementioned substituents, and a covalent bond is present between a hydrogen atom and a carbon atom of the dehydration target.

The dehydrogenation reaction catalyst of the present embodiment may also be used for promoting a dehydrogenation reaction other than ALKANE oxidative coupling reaction. Examples of another dehydrogenation reaction which the dehydrogenation reaction catalyst of the present embodiment promotes include a dehydrogenation reaction of ALKANE as exemplified in formula (23) of FIG. 4, and a dehydrogenation reaction of an alcohol or an alcohol derivative (hereinafter may also be collectively referred to as "ALCOHOL") as exemplified in formulas (24) and (25). In formulas (23) to (25), each of A and B represents hydrogen, or an atom or a group substituting for hydrogen, similar to the cases of formulas (13) to (22). Formula (23) represents a reaction for forming an alkene or an alkene derivative from ALKANE. Formula (24) represents a reaction for forming an aldehyde from ALCOHOL. Formula (25) represents a reaction for forming a ketone from ALCOHOL. As described above, the dehydrogenation reaction catalyst of the present embodiment may be used as a catalyst for promoting various dehydrogenation reactions.

[Dehydrogenation reaction catalyst production method]

The dehydrogenation reaction catalyst of the present embodiment may be produced through, for example, a solid-phase reaction method. In a specific procedure, powders of metal oxides or metal carbonates serving as raw materials of the perovskite-type oxide represented by formula (26) are mixed together with a solvent such as ethanol by means of a ball mill or the like. Then, the mixture dried to remove the solvent and fired. When firing is conducted at relatively high temperature (about 1,300 to 1,600°C), there can be yielded a complex oxide represented by formula (26). As a method for producing the complex oxide, there have been known a variety of methods such as a solid-phase reaction method, a complex polymerization method, a co-precipitation method, and a sol-gel method. Thus, the complex oxide may be produced through any of these methods.

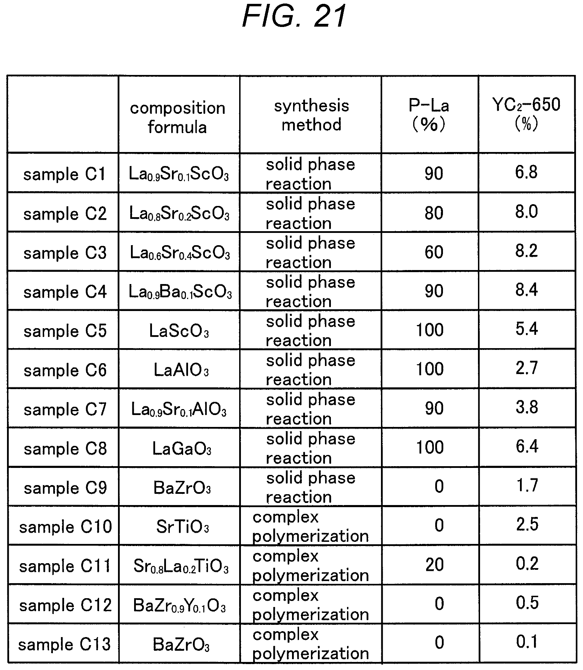

FIG. 21 is a sixth table showing a $C_2$ yield obtained by dehydrogenation reaction catalysts of the present embodiment. FIG. 21 shows the results investigation of the performance of 12 complex oxides of samples C1 to C12. Among samples C1 to C12, samples C1 to C8 are perovskite-type oxides satisfying the conditions: the aforementioned formula (26), the type and proportions of the elements present in A sites, the type of elements present in B sites, and the oxygen deficiency. Samples 9 to C12 are complex oxides not satisfying at least one condition among the aforementioned formula (26), the type and proportions of the elements present in A sites, the type of elements present in B sites, and the oxygen deficiency. In FIG. 21, the lanthanum proportion of the A sites in the perovskite-type oxide of each of samples C1 to C12 is denoted by "P-La." Notably, in the following description and drawings, the number of oxygen atoms in any formula may be denoted simply as "$O_3$" in some cases. However, the denotation is equivalent to "$O_{3-\delta}$" and does not mean only the case in which $\delta$ is strictly 0. Next, the composition, production method, evaluation method, and results of performance evaluation with respect to the samples will be described. In the following description, the methane oxidative coupling catalyst was evaluated on the basis of the performance intrinsic to a dehydrogenation reaction catalyst.

[Production of sample C1]

A catalyst ($La_{0.9}Sr_{0.1}ScO_3$) of sample C1 was prepared through a solid phase reaction method. As raw material powders, lanthanum hydroxide (product of Shin-Etsu Chemical Co., Ltd.), strontium carbonate (product of Kojundo Chemical Laboratory Co., Ltd.), and scandium oxide (product of Alfa Aesar) were used. These raw material powders were weighed so that the proportion of metal elements were adjusted to establish the composition of a compositional formula $La_{0.9}Sr_{0.1}ScO_3$. To the mixture, boulders (diameter: 5 mm) and ethanol were added, and the resultant mixture was sufficiently mixed by means of a ball mill for 15 hours. Thereafter, an ethanol component was evaporated, to thereby yield a solid. The solid was sieved through a sieve (opening: 250 μm) and, then fired at 1,300°C for 5 hours. To the thus-obtained powder, boulders (diameter: 5 mm) and ethanol were added, and pulverization was conducted again by means of a ball mill for 15 hours with mixing. Thereafter, an ethanol component was evaporated, to thereby yield a solid. The solid was sieved through a sieve (opening: 250 μm), to thereby yield a dehydrogenation reaction catalyst as sample C1.

[Production of samples C2 to C9]

Powder-form dehydrogenation reaction catalysts as samples C2 to C9 were produced in a manner similar to production of sample C1, except that the type and weighing amount of a catalyst raw material powder were changed to attain the compositional proportions of the compositional formulas of samples C2 to C9 shown in FIG. 21. In the cases of samples C3 and C4, the heat treatment temperature in the firing step was modified to fall within a range of 1,300°C to 1,350°C, so as to attain high target substance content. For example, the temperature was 1,400°C in the case of sample C3, and the temperature was 1,350°C in the case of sample C4. Thus, the heat treatment temperature during the firing step in production of the samples may be modified so as to attain high target substance content. In order to add, as constituent elements, lanthanum, strontium, scandium, barium, aluminum, gallium, and zirconium, to a dehydrogenation reaction catalyst, there were used the following raw material powders: lanthanum hydroxide (product of Shin-Etsu Chemical Co., Ltd.), strontium carbonate (product of Kojundo Chemical Laboratory Co., Ltd.), scandium oxide (product of Alfa Aesar), barium carbonate (product of Sakai Chemical Industry Co., Ltd.), aluminum oxide (product of Sumitomo Chemical Co., Ltd.), gallium oxide (product of Kishida Chemical Co., Ltd.), and zirconium oxide (product of Daiichi Kigenso Kagaku Kogyo Co., Ltd.).

[Production of sample C10]

A catalyst ($SrTiO_3$) of sample C10 was prepared through a complex polymerization method. As raw material powders, strontium nitrate (product of FUJIFILM Wako Pure Chemical Corporation) and tetra-i-propoxytitanium (product of Kojundo Chemical Laboratory Co., Ltd.) were used. These raw material powders were weighed so that the proportion of metal elements were adjusted to establish the composition of a compositional formula $SrTiO_3$. To the mixture of the above raw material powders, aqueous citric acid and propylene glycol were added so that the proportions: metal element : citric acid : propylene glycol were adjusted to 1:3:3 (by mole). The resultant mixture was agitated and sufficiently mixed at 80°C for 1 hour. The resultant solution was slowly heated to 300°C, to thereby yield a polymer in which the metals were dispersed. The thus-obtained polymer was heated at 400°C for 2 hours, and the formed carbonized product was pulverized by means of an agate mortar, to thereby yield a precursor of a catalyst powder. The thus-obtained catalyst powder precursor was subjected to a heat treatment at 1,200°C for 6 hours, to thereby yield a powder-form dehydrogenation reaction catalyst as sample C10.

[Production of samples C11 to C13]

Powder-form dehydrogenation reaction catalysts as samples C11 to C13 were produced in a manner similar to production of sample C10, except that the type and weighing amount of a catalyst raw material powder were changed to attain the compositional proportions of the compositional formulas of samples C11 to C13 shown in FIG. 21. In order to add, as constituent elements, strontium, lanthanum, titanium, barium, zirconium, and yttrium, to a dehydrogenation reaction catalyst, there were used the following raw material powders: strontium nitrate (product of FUJIFILM Wako Pure Chemical Corporation), lanthanum nitrate (product of FUJIFILM Wako Pure Chemical Corporation), tetra-i-propoxytitanium (product of Kojundo Chemical Laboratory Co., Ltd.), barium nitrate (product of FUJIFILM Wako Pure Chemical Corporation), zirconyl nitrate (product of FUJIFILM Wako Pure Chemical Corporation), yttrium nitrate (product of Sigma Aldrich)

[Determination of $C_2$ yield]

Each of samples C1 to C13 was weighed to take a portion of 0.1 g and placed in an immobilized bed flow reactor. While the reactor was heated at 750°C, a gas mixture containing methane, oxygen, and nitrogen was caused to flow through the reactor at a pressure of 1 atm, a flow rate of 45 $cm^3$/min, and flow proportions $CH_4$:$O_2$:$N_2$ of 3.8:1:4. Under the conditions, a catalytic activity test was conducted. The catalytic activity test was carried out, while the sample was heated at 650°C. The compositions of the gas fed to the apparatus employed in the catalytic activity test and the gas exhausted from the apparatus were analyzed by means of a microgas chromatograph (3000A, product of Agilent). The $C_2$ yield at 650°C was calculated on the basis of the compositional analysis ("YC$_2$-650," in FIG. 21).

As shown in FIG. 21, among samples C1 to C13, samples C1 to C8; i.e., complex oxides each containing a relatively large amount of lanthanum in A sites, were found to exhibit a $C_2$ yield at 650°C (YC$_2$-650) greater than that of samples C1 9o C13; i.e., complex oxides each containing only a small amount of lanthanum in A sites. A complex oxide containing a relatively large amount of lanthanum in A sites is a basic oxide. Thus, such complex oxides were found to exhibit an enhanced catalytic activity intrinsic to a dehydrogenation reaction catalyst at a relatively low temperature of 650°C.

Also, among samples C1 to C8, samples C1 to C4 each have A sites in which strontium or barium partially substituted by lanthanum. Thus, in samples C1 to C4, the basicity intrinsic to a dehydrogenation reaction catalyst and the oxygen deficiency increase. In a methane oxidative coupling reaction at a high temperature of 700°C or higher, motion of molecules of $CO_2$ formed as a by-product during the methane oxidative coupling reaction is activated. Thus, the effect provided by adsorption of $CO_2$ molecules is minimized regardless of the material of the catalyst. Accordingly, a complex oxide containing strontium or barium is useful as a catalyst for methane oxidative coupling reaction. However, the temperature is lower than 700°C (e.g., 650°C), molecules of $CO_2$, a by-product during the methane oxidative coupling reaction, is readily adsorbed on a catalyst, possibly evoking poisoning of the catalyst resulting in deactivation, since strontium and barium, which are alkaline earth metals, are elements readily forming a carbonate salt. In contrast, samples C1 to C4 each have A sites in which strontium or barium partially substituted by lanthanum, which impedes formation of a carbonate salt. In such a case, formation of a carbonate salt is impeded more effectively in the case of the above dehydrogenation reaction catalyst than in the case of a complex oxide containing strontium or barium. Thus, conceivably, the effect of adsorption of $CO_2$ molecules at 650°C is reduced, thereby suppressing deactivation.

Furthermore, as in the case of sample C11, the catalytic activity intrinsic to a dehydrogenation reaction catalyst was found to decrease when lanthanum was incorporated into $SrTiO_3$. Thus, only a small amount of added lanthanum was found to be insufficient for enhancing catalytic activity without being affected by adsorption of $CO_2$ molecules. When the amount of lanthanum added is very small, conceivably, the lower basicity of lanthanum as compared with strontium predominates, thereby lowering catalytic activity. Thus, the effect of lanthanum on suppressing adsorption of $CO_2$ molecules requires a large amount of addition of lanthanum.

In the case of sample C11 in which tetravalent elements are present in B sites, substitution by lanthanum may possibly insufficient. Therefore, a dehydrogenation reaction catalyst including a perovskite-type oxide in which trivalent elements are present in B sites and lanthanum is introduced in A sites (e.g., the cases of samples C1 to C8) exhibits excellent methane oxidative coupling reactivity, as compared with a dehydrogenation reaction catalyst including a perovskite-type oxide in which tetravalent elements are present in B sites (e.g., the case of sample C11). Also, sample C3, having a lanthanum substitution ratio in A sites of 0.6, was found to evoke excellent methane oxidative coupling reaction when sample C3 serves as a dehydrogenation reaction catalyst. Thus, the dehydrogenation reaction catalyst can evoke excellent methane oxidative coupling reaction by tuning the lanthanum substitution ratio in A sites to at least 0.4 or more.

The dehydrogenation reaction catalyst of the present embodiment having the aforementioned technical features includes a perovskite-type oxide containing lanthanum in A sites. As a result, the catalytic activity intrinsic to a dehydrogenation reaction catalyst; e.g., promoting dehydrogenation reaction at low temperature, can be enhanced.

In addition to lanthanum, the dehydrogenation reaction catalyst of the present embodiment also contains strontium or barium in A sites. As a result, among catalytic activities intrinsic to a dehydrogenation reaction catalyst, the catalytic activity of promoting dehydrogenation reaction at relatively low temperature can be enhanced.

When the dehydrogenation reaction catalyst of the present embodiment is employed as a methane oxidative coupling catalyst, the catalytic activity of the methane oxidative coupling catalyst at low temperature can be enhanced.

<Variations of the present embodiment>

The present invention is not limited to the aforementioned specific embodiments, and may be carried out in various modes, so long as they do not deviate from the gist of the invention. For example, the following variation may be encompassed in the invention.

[Variation 1]

In the first embodiment, the dehydrogenation reaction catalyst exhibiting a ratio of a second peak area to a first peak area greater than 1 in an O1s orbital spectrum included in a photoelectron spectrum obtained through X-ray photoelectron spectroscopy is a complex oxide which includes a primary phase having a perovskite-type oxide crystal structure represented by formula (1) and a secondary phase containing at least one member of three complex oxides represented by formulas (2a) to (2c). However, the composition of the dehydrogenation reaction catalyst exhibiting a ratio of a second peak area to a first peak area greater than 1 is not limited to the above composition.

The present disclosure is not limited to the aforementioned embodiments and the like, and may be carried out in various modes, so long as they do not deviate from the gist of the invention. For example, technical features of each of the embodiments corresponding to technical features of each of the modes described in "SUMMARY OF INVENTION" may be appropriately replaced or combined in order to solve the entirety or a part of the aforementioned problems or to attain the entirety or a part of the aforementioned effects. Unless described as essential features in the present specification, the technical features may be appropriately deleted.

The present disclosure may be realized in the following modes.

[Application Example 1]

A dehydrogenation reaction catalyst, characterized by including:

a primary phase formed of a perovskite-type oxide represented by a general formula $(A_{1-x}A'_x)(Zr_{1-y-z}B_yB'_z)O_{3-\delta}$ (wherein A represents at least one element selected from alkaline earth metals; A' represents at least one element of lanthanum (La) and yttrium (Y); B represents at least one element of titanium (Ti) and cerium (Ce); B' represents at least one element selected from among yttrium (Y), scandium (Sc), ytterbium (Yb), aluminum (Al), indium (In), and neodymium (Nd); relationships: $0 \leq x \leq 0.4$, $0.3 \leq (1-z) \leq 1$, $0 \leq y$, and $0 < (1-y-z)$ are satisfied; and $\delta$ represents an oxygen

deficiency), and
a secondary phase formed of at least one member of three complex oxides represented by general formulas $AB'_2O_4$, $A_2B'_2O_5$, and $A_3B'_4O_9$, respectively (wherein A and B' are the same elements as A and B' forming the perovskite-type oxide).

[Application Example 2]

A dehydrogenation reaction catalyst as described in Application Example 1, characterized in that

A is barium (Ba), B' is scandium (Sc), and
the secondary phase is formed of a barium scandate (at least one member of three complex oxides represented by formulas $BaSc_2O_4$, $Ba_2Sc_2O_5$, and $Ba_3Sc_4O_9$).

[Application Example 3]

A dehydrogenation reaction catalyst as described in Application Example 1 or 2, characterized in that the secondary phase is formed of $Ba_3Sc_4O_9$.

[Application Example 4]

A dehydrogenation reaction catalyst as described in any one of Application Examples 1 to 3, characterized in that: the primary phase is formed of $BaZr_{1-z}Sc_zO_{3-\delta}$ (wherein a relationship $0.1 \leq z \leq 0.7$ is satisfied).

[Application Example 5]

A dehydrogenation reaction catalyst as described in any one of Application Examples 1 to 4, characterized in that, in a powder X-ray diffraction pattern obtained by a CuKα ray, the perovskite-type oxide forming the primary phase exhibits a first peak having a highest peak intensity within a diffraction angle range 2θ of 29.5 to 30.5°, and the complex oxide forming the secondary phase exhibits a second peak having a highest peak intensity within a diffraction angle range 2θ of 30.6 to 31.0°, in which the ratio in intensity of the second peak to the first peak is 0.04 or greater.

[Application Example 6]

A dehydrogenation reaction catalyst as described in any one of Application Examples 1 to 5, characterized in that the secondary phase is formed of at least one member of the three complex oxides.

[Application Example 7]

A dehydrogenation reaction catalyst as described in any one of Application Examples 1 to 6, characterized in that the dehydrogenation reaction catalyst is a methane oxidative coupling catalyst for producing a C≥2 hydrocarbon from methane.

[Application Example 8]

A dehydrogenation reaction catalyst, characterized in that

when an O1s orbital spectrum included in a photoelectron spectrum obtained through X-ray photoelectron spectroscopy is split, through a peak separation fitting processing, to a first curve including a first peak attributed to a maximum value of binding energy within a range of 525 to 530 eV, and a second curve including a second peak attributed to a maximum value of binding energy within a range of 530 to 535 eV, and
an area of a convex part including the first peak in the first curve is defined as a first peak area, and an area of a convex part including the second peak in the second curve is defined as a second peak area,
a ratio of the second peak area to the first peak area is greater than 1.

[Application Example 9]

A dehydrogenation reaction catalyst as described in any one of Application Examples 1 to 8, characterized in that the dehydrogenation reaction catalyst has oxygen deficiency.

[Application Example 10]

A dehydrogenation reaction catalyst as described in any one of Application Examples 1 to 9, characterized in that the primary phase included in the catalyst has a perovskite structure.

[Application Example 11]

A dehydrogenation reaction catalyst as described in any one of Application Examples 1 to 10, characterized in that

the dehydrogenation reaction catalyst has a specific surface area as determined through the BET technique is 10 m$^2$/g or more.

[Application Example 12]

A dehydrogenation reaction catalyst as described in any one of Application Examples 1 to 11, characterized in that

the dehydrogenation reaction catalyst is a methane oxidative coupling catalyst for producing a C≥2 hydrocarbon from methane.

[Application Example 13]

A dehydrogenation reaction catalyst characterized by

having a perovskite structure represented by a general formula $(La_{1-x}M1_x)M2O_{3-\delta}$ (wherein M1 represents at least one element selected from alkaline earth metals; M2 represents at least one element selected from among yttrium (Y), scandium (Sc), ytterbium (Yb), aluminum (Al), indium (In), and gallium (Ga); $\delta$ represents an oxygen deficiency; and a relationship $0 \leq x \leq 0.6$ is satisfied).

[Application Example 14]

A dehydrogenation reaction catalyst as described in any one of Application Examples 1 to 13, characterized in that

M1 is strontium (Sr) or barium (Ba).

[Application Example 15]

A dehydrogenation reaction catalyst as described in any one of Application Examples 1 to 14, characterized in that

the dehydrogenation reaction catalyst is a methane oxidative coupling catalyst for producing a C≥2 hydrocarbon from methane.

**Claims**

1. A dehydrogenation reaction catalyst, **characterized by** comprising:

a primary phase formed of a perovskite-type oxide represented by a general formula $(A_{1-x}A'_x)(Zr_{1-y-z}B_yB'_z)O_{3-\delta}$ (wherein A represents at least one element selected from alkaline earth metals; A' represents at least one element of lanthanum (La) and yttrium (Y); B represents at least one element of titanium (Ti) and cerium (Ce); B' represents at least one element selected from among yttrium (Y), scandium (Sc), ytterbium (Yb), aluminum (Al), indium (In), and neodymium (Nd); relationships: $0 \leq x \leq 0.4$, $0.3 \leq (1-z) \leq 1$, $0 \leq y$, and $0 < (1-y-z)$ are satisfied; and $\delta$ represents an oxygen deficiency), and
a secondary phase formed of at least one member of three complex oxides represented by general formulas $AB'_2O_4$, $A_2B'_2O_5$, and $A_3B'_4O_9$, respectively (wherein A and B' are the same elements as A and B' forming the perovskite-type oxide).

2. The dehydrogenation reaction catalyst according to claim 1, wherein

A is barium (Ba), and

B' is scandium (Sc), and
the secondary phase is formed of a barium scandate (at least one member of three complex oxides represented by formulas $BaSc_2O_4$, $Ba_2Sc_2O_5$, and $Ba_3Sc_4O_9$).

3. The dehydrogenation reaction catalyst according to claim 2, wherein
the secondary phase is formed of $Ba_3Sc_4O_9$.

4. The dehydrogenation reaction catalyst according to claim 3, wherein
the primary phase is formed of $BaZr_{1-z}Sc_zO_{3-\delta}$ (wherein a relationship $0.1 \leq z \leq 0.7$ is satisfied).

5. The dehydrogenation reaction catalyst according to claim 4, wherein,

in a powder X-ray diffraction pattern obtained by a $CuK\alpha$ ray,
the perovskite-type oxide forming the primary phase exhibits a first peak having a highest peak intensity within a diffraction angle range $2\theta$ of 29.5 to 30.5°, and the complex oxide forming the secondary phase exhibits a second peak having a highest peak intensity within a diffraction angle range $2\theta$ of 30.6 to 31.0°,
wherein the ratio in intensity of the second peak to the first peak is 0.04 or greater.

6. The dehydrogenation reaction catalyst according to claim 1 or 2, wherein
the secondary phase is formed of any one member of the three complex oxides.

7. The dehydrogenation reaction catalyst according to claim 1 or 2,
which is a methane oxidative coupling catalyst for producing a C≥2 hydrocarbon from methane.

8. A dehydrogenation reaction catalyst, **characterized in that**

when an O1s orbital spectrum included in a photoelectron spectrum obtained through X-ray photoelectron spectroscopy is split, through a peak separation fitting processing, to a first curve including a first peak attributed to a maximum value of binding energy within a range of 525 to 530 eV, and a second curve including a second peak attributed to a maximum value of binding energy within a range of 530 to 535 eV, and
an area of a convex part including the first peak in the first curve is defined as a first peak area, and an area of a convex part including the second peak in the second curve is defined as a second peak area,
a ratio of the second peak area to the first peak area is greater than 1.

9. The dehydrogenation reaction catalyst according to claim 8,
which has oxygen deficiency.

10. The dehydrogenation reaction catalyst according to claim 8 or 9, wherein
the primary phase included in the catalyst has a perovskite structure.

11. The dehydrogenation reaction catalyst according to claim 8 or 9,
which has a specific surface area as determined through the BET technique is 10 $m^2/g$ or more.

12. The dehydrogenation reaction catalyst according to claim 8 or 9,
which is a methane oxidative coupling catalyst for producing a C≥2 hydrocarbon from methane.

13. A dehydrogenation reaction catalyst **characterized by**
having a perovskite structure represented by a general formula $(La_{1-x}M1_x)M2O_{3-\delta}$ (wherein M1 represents at least one element selected from alkaline earth metals; M2 represents at least one element selected from among yttrium (Y), scandium (Sc), ytterbium (Yb), aluminum (Al), indium (In), and gallium (Ga); $\delta$ represents an oxygen deficiency; and a relationship $0 \leq x \leq 0.6$ is satisfied).

14. The dehydrogenation reaction catalyst according to claim 13, wherein
M1 is strontium (Sr) or barium (Ba).

15. The dehydrogenation reaction catalyst according to claim 13 or 14,
which is a methane oxidative coupling catalyst for producing a C≥2 hydrocarbon from methane.

EP 4 640 310 A1

## FIG. 1

# FIG. 2

OXIDATION SITE
$$CH_4 \rightarrow \cdot CH_3 + H^+ + e^- \quad \cdots (5)$$

REDUCTION SITE
$$1/2 O_2 + 2H^+ + 2e^- \rightarrow H_2O \quad \cdots (7)$$

CH₄  O₂

$H^+$  $H^+$

$e^-$  $e^-$

C₂H₄  H₂O

NEAR CATALYST SURFACE (GAS PHASE REACTION)
$$2 \cdot CH_3 + 1/2 O_2 \rightarrow C_2H_4 + H_2O \quad \cdots (6)$$

# FIG. 3

OXIDATION SITE
$CH_4 \rightarrow \cdot CH_3 + H^+ + e^- \quad \cdots(5)$

REDUCTION SITE
$O_2 + 4H^+ + 4e^- \rightarrow 2H_2O \quad \cdots(9)$

NEAR CATALYST SURFACE (GAS PHASE REACTION)
$2 \cdot CH_3 \rightarrow C_2H_6 \rightarrow C_2H_4 + 2H^+ + 2e^- \cdots(8)$

# FIG. 4

$$CH_4 + CH_4 \rightarrow H_2C{=}CH_2 \quad \cdots \quad (11)$$

$$CH_4 + CH_4 \rightarrow -\!\!\left\{ CH_2 - CH_2 \right\}_n \quad \cdots \quad (12)$$

$$BACH_2 + BACH_2 \rightarrow BAC{=}CAB \quad \cdots \quad (13)$$

$$BACH_2 + BACH_2 \rightarrow -\!\!\left\{ CAB - CAB \right\}_n \quad \cdots \quad (14)$$

$$ACH_3 + ACH_3 \rightarrow HAC{=}CAH \quad \cdots \quad (15)$$

$$ACH_3 + ACH_3 \rightarrow -\!\!\left\{ CAH - CAH \right\}_n \quad \cdots \quad (16)$$

$$CH_4 + CAH_3 \rightarrow H_2C{=}CAH \quad \cdots \quad (17)$$

$$CH_4 + CAH_3 \rightarrow -\!\!\left\{ CH_2 - CAH \right\}_n \quad \cdots \quad (18)$$

$$CAH_3 + CA_2H_2 \rightarrow HAC{=}CA_2 \quad \cdots \quad (19)$$

$$CAH_3 + CA_2H_2 \rightarrow -\!\!\left\{ CAH - CA_2 \right\}_n \quad \cdots \quad (20)$$

$$CH_4 + CABH_2 \rightarrow H_2C{=}CAB \quad \cdots \quad (21)$$

$$CH_4 + CABH_2 \rightarrow -\!\!\left\{ CH_2 - CAB \right\}_n \quad \cdots \quad (22)$$

$$CH_2ACH_2B \rightarrow HAC{=}CBH \quad \cdots \quad (23)$$

$$ACH_2OH \rightarrow ACH{=}O \quad \cdots \quad (24)$$

$$ABCHOH \rightarrow ABC{=}O \quad \cdots \quad (25)$$

## FIG. 5

| | composition formula | T_treat (°C) | C_total (S/cm) | TN_pro (—) | TN_e/h (—) | C_pro (S/cm) | YC$_2$-750 (%) |
|---|---|---|---|---|---|---|---|
| sample A1 | $BaZr_{0.8}Sc_{0.2}O_3$ | 1200 | 3.8E-03 | 0.605 | 0.36 | 2.3E-03 | 15.2 |
| sample A2 | $BaZr_{0.6}Sc_{0.4}O_3$ | 1200 | 4.2E-03 | 0.619 | 0.34 | 2.6E-03 | 15.9 |
| sample A3 | $BaZr_{0.4}Sc_{0.6}O_3$ | 1200 | 1.9E-03 | 0.526 | 0.43 | 1.0E-03 | 16.6 |
| sample A4 | $BaZr_{0.3}Sc_{0.7}O_3$ | 1200 | 4.7E-04 | 0.745 | 0.22 | 3.5E-04 | 13.8 |
| sample A5 | $BaZrO_3$ | 1300 | 2.2E-05 | 0.132 | 0.82 | 2.9E-06 | 9.4 |
| sample A6 | $BaZr_{0.8}Y_{0.2}O_3$ | 1300 | 3.7E-03 | 0.595 | 0.38 | 2.2E-03 | 14.7 |
| sample A7 | $BaZr_{0.6}Y_{0.4}O_3$ | 1300 | 2.3E-04 | 0.739 | 0.22 | 1.7E-04 | 15.6 |
| sample A8 | $BaZr_{0.6}Yb_{0.4}O_3$ | 1200 | 2.8E-03 | 0.571 | 0.34 | 1.6E-03 | 14.2 |
| sample A9 | $BaZr_{0.6}In_{0.4}O_3$ | 1200 | 1.9E-03 | 0.579 | 0.39 | 1.1E-03 | 15.0 |
| sample A10 | $BaZr_{0.6}Nd_{0.4}O_3$ | 1200 | 1.5E-03 | 0.640 | 0.33 | 9.6E-04 | 13.9 |
| sample A11 | $BaZr_{0.6}Y_{0.2}Yb_{0.2}O_3$ | 1200 | 2.2E-03 | 0.682 | 0.29 | 1.5E-03 | 14.4 |
| sample A12 | $BaZr_{0.4}Ce_{0.4}Y_{0.2}O_3$ | 1200 | 1.7E-03 | 0.765 | 0.21 | 1.3E-03 | 13.4 |
| sample A13 | $Ba_{0.8}La_{0.2}ZrO_3$ | 1200 | 3.3E-05 | 0.079 | 0.91 | 2.6E-06 | 7.5 |
| sample A14 | $Ba_{0.8}Y_{0.2}ZrO_3$ | 1200 | 2.9E-05 | 0.086 | 0.90 | 2.5E-06 | 6.7 |
| sample A15 | $Ba_{0.8}La_{0.2}Zr_{0.8}Y_{0.2}O_3$ | 1200 | 6.2E-03 | 0.158 | 0.83 | 9.8E-03 | 12.8 |
| sample A16 | $Ba_{0.8}Sr_{0.2}Zr_{0.8}Y_{0.2}O_3$ | 1200 | 4.3E-03 | 0.464 | 0.50 | 2.0E-03 | 13.0 |
| sample A17 | $Ba_{0.6}Sr_{0.4}Zr_{0.8}Y_{0.2}O_3$ | 1200 | 8.4E-03 | 0.252 | 0.73 | 2.2E-03 | 11.5 |

FIG. 6

| | composition formula | T_treat (°C) | C_total (S/cm) | TN_pro (—) | TN_e/h (—) | C_pro (S/cm) | YC$_2$-750 (%) |
|---|---|---|---|---|---|---|---|
| sample A18 | $CaZrO_3$ | 1200 | 3.8E-05 | 0.037 | 0.95 | 1.4E-06 | 8.1 |
| sample A19 | $CaZr_{0.8}Y_{0.2}O_3$ | 1200 | 7.6E-04 | 0.803 | 0.20 | 6.1E-04 | 10.9 |
| sample A20 | $CaZr_{0.8}Sc_{0.2}O_3$ | 1200 | 8.0E-04 | 0.825 | 0.17 | 6.6E-04 | 12.8 |
| sample A21 | $SrZrO_3$ | 1200 | 8.2E-05 | 0.032 | 0.96 | 2.6E-06 | 8.8 |
| sample A22 | $SrZr_{0.8}Y_{0.2}O_3$ | 1200 | 8.8E-04 | 0.0875 | 0.12 | 7.7E-04 | 11.4 |
| sample A23 | $SrZr_{0.8}Sc_{0.2}O_3$ | 1200 | 9.1E-04 | 0.857 | 0.14 | 7.8E-04 | 13.0 |
| sample A24 | $Sr_{0.8}Ca_{0.2}Zr_{0.8}Y_{0.2}O_3$ | 1200 | 6.2E-04 | 0.887 | 0.11 | 5.5E-04 | 11.2 |
| sample A25 | $Sr_{0.8}La_{0.2}ZrO_3$ | 1200 | 9.7E-04 | 0.002 | 0.99 | 1.8E-06 | 6.9 |
| sample A26 | $Sr_{0.8}La_{0.2}Zr_{0.8}Y_{0.2}O_3$ | 1200 | 9.9E-04 | 0.444 | 0.55 | 4.4E-04 | 10.0 |
| sample A27 | $BaZr_{0.25}Ce_{0.05}Sc_{0.7}O_3$ | 1200 | 4.8E-04 | 0.750 | 0.25 | 3.6E-04 | 13.5 |
| sample A28 | $BaZr_{0.25}Ti_{0.05}Sc_{0.7}O_3$ | 1200 | 2.9E-04 | 0.655 | 0.34 | 1.9E-04 | 13.1 |
| sample A29 | $BaZr_{0.1}Ce_{0.1}Ti_{0.1}Sc_{0.7}O_3$ | 1200 | 2.2E-04 | 0.727 | 0.27 | 1.6E-04 | 13.4 |
| sample A30 | $BaZr_{0.25}Sc_{0.75}O_3$ | 1200 | 8.9E-05 | 0.371 | 0.57 | 3.3E-05 | 4.9 |
| sample A31 | $BaZr_{0.25}Y_{0.75}O_3$ | 1200 | 1.6E-06 | 0.425 | 0.52 | 1.0E-06 | 2.6 |
| sample A32 | $Ba_{0.5}La_{0.5}ZrO_3$ | 1200 | 8.2E-05 | 0.099 | 0.88 | 8.1E-06 | 2.2 |
| sample A33 | $BaZr_{0.6}Fe_{0.4}O_3$ | 1200 | 6.6E-06 | 0.303 | 0.56 | 2.0E-06 | 3.1 |
| sample A34 | $Sr_{0.8}La_{0.2}TiO_3$ | 1000 | 1.3E-01 | 0.000 | 0.99 | 3.1E-05 | 5.8 |

EP 4 640 310 A1

*FIG. 7*

## FIG. 8

EP 4 640 310 A1

## FIG. 9

| | composition formula | synthesis method | T_treat (°C) | YC$_2$–750 (%) | YC$_2$–800 (%) | $\Delta$YC$_2$ (%) | R_peak intensity (—) |
|---|---|---|---|---|---|---|---|
| sample A3 | BaZr$_{0.4}$Sc$_{0.6}$O$_3$ | complex polymerization | 1200 | 16.6 | 14.2 | 14.46 | 0.01163 |
| sample A35 | BaZr$_{0.4}$Sc$_{0.6}$O$_3$ | solid phase reaction | 1300 | 15.5 | 15.3 | 1.29 | 0.05748 |

## FIG. 10

FIG. 11

| | composition formula | synthesis method | T_treat (°C) | $YC_2$–750 (%) | $YC_2$–800 (%) | $\Delta YC_2$ (%) | R_peak intensity (—) |
|---|---|---|---|---|---|---|---|
| sample A36 | $BaZr_{0.6}Sc_{0.4}O_3$ | solid phase reaction | 1300 | 14.3 | 12.4 | −1.29 | 0.04861 |
| sample A35 | $BaZr_{0.4}Sc_{0.6}O_3$ | solid phase reaction | 1300 | 15.5 | 15.3 | 1.29 | 0.05748 |
| sample A37 | $BaZr_{0.35}Sc_{0.65}O_3$ | solid phase reaction | 1300 | 14.9 | 13.1 | 1.14 | 0.14447 |
| sample A38 | $BaZr_{0.2}Sc_{0.8}O_3$ | solid phase reaction | 1300 | 13.2 | 11.0 | −0.57 | 0.42805 |
| sample A3 | $BaZr_{0.4}Sc_{0.6}O_3$ | complex polymerization | 1200 | 16.6 | 14.2 | 14.46 | 0.01163 |
| sample A39 | $Ba_3Sc_4O_9$ | solid phase reaction | 1300 | 6.5 | 6.1 | −57.86 | 282.11897 |

EP 4 640 310 A1

## FIG. 12

sample B1

x30,000　5.0kV　UED　SEM　WD 4.0mm　100nm

# FIG. 13

sample B12

## FIG. 14

| | composition formula | synthesis method | Specific surface area $(m^2/g)$ |
|---|---|---|---|
| sample B1 | $BaZr_{0.8}Y_{0.2}O_3$ | solid phase reaction | 12.5 |
| sample B2 | $BaZr_{0.6}Y_{0.4}O_3$ | solid phase reaction | 10.2 |
| sample B3 | $BaZr_{0.8}Sc_{0.2}O_3$ | solid phase reaction | 13.9 |
| sample B4 | $BaZr_{0.6}Sc_{0.4}O_3$ | solid phase reaction | 13.4 |
| sample B5 | $BaZr_{0.4}Sc_{0.6}O_3$ | solid phase reaction | 12.3 |
| sample B12 | $BaZr_{0.8}Y_{0.2}O_3$ | complex polymerization | 5.7 |

## FIG. 15

FIG. 16

# FIG. 17

| | composition formula | synthesis method | Sao/Slo (−) | $YC_2$−700 (%) |
|---|---|---|---|---|
| sample B1 | $BaZr_{0.8}Y_{0.2}O_3$ | solid phase reaction | 1.89 | 10.5 |
| sample B2 | $BaZr_{0.6}Y_{0.4}O_3$ | solid phase reaction | 5.82 | 10.1 |
| sample B3 | $BaZr_{0.8}Sc_{0.2}O_3$ | solid phase reaction | 1.67 | 12.2 |
| sample B4 | $BaZr_{0.6}Sc_{0.4}O_3$ | solid phase reaction | 1.64 | 12.4 |
| sample B5 | $BaZr_{0.4}Sc_{0.6}O_3$ | solid phase reaction | 4.26 | 12.6 |
| sample B6 | $BaZr_{0.35}Sc_{0.65}O_3$ | solid phase reaction | 2.96 | 13.1 |
| sample B7 | $BaZr_{0.2}Sc_{0.8}O_3$ | solid phase reaction | 3.97 | 11.0 |
| sample B8 | $La_{0.9}Sr_{0.1}ScO_3$ | solid phase reaction | 1.59 | 9.2 |
| sample B9 | $La_{0.8}Sr_{0.2}ScO_3$ | solid phase reaction | 1.17 | 10.5 |
| sample B10 | $La_{0.6}Sr_{0.4}ScO_3$ | solid phase reaction | 2.45 | 11.2 |
| sample B11 | $La_{0.9}Ba_{0.1}ScO_3$ | solid phase reaction | 1.77 | 10.7 |
| sample B12 | $BaZr_{0.8}Y_{0.2}O_3$ | complex polymerization | 1.00 | 8.4 |
| sample B13 | $BaZrO_3$ | solid phase reaction | 0.86 | 6.1 |

# FIG. 18

## FIG. 19

# FIG. 20

| | composition formula | synthesis method | YC$_2$-700 (%) | YC$_2$-750 (%) |
|---|---|---|---|---|
| sample B4 | BaZr$_{0.6}$Sc$_{0.4}$O$_3$ | solid phase reaction | 12.4 | 14.3 |
| sample B5 | BaZr$_{0.4}$Sc$_{0.6}$O$_3$ | solid phase reaction | 12.6 | 15.5 |
| sample B6 | BaZr$_{0.35}$Sc$_{0.65}$O$_3$ | solid phase reaction | 13.1 | 14.9 |
| sample B7 | BaZr$_{0.2}$Sc$_{0.8}$O$_3$ | solid phase reaction | 11.0 | 13.2 |

## FIG. 21

| | composition formula | synthesis method | P-La (%) | YC$_2$-650 (%) |
|---|---|---|---|---|
| sample C1 | La$_{0.9}$Sr$_{0.1}$ScO$_3$ | solid phase reaction | 90 | 6.8 |
| sample C2 | La$_{0.8}$Sr$_{0.2}$ScO$_3$ | solid phase reaction | 80 | 8.0 |
| sample C3 | La$_{0.6}$Sr$_{0.4}$ScO$_3$ | solid phase reaction | 60 | 8.2 |
| sample C4 | La$_{0.9}$Ba$_{0.1}$ScO$_3$ | solid phase reaction | 90 | 8.4 |
| sample C5 | LaScO$_3$ | solid phase reaction | 100 | 5.4 |
| sample C6 | LaAlO$_3$ | solid phase reaction | 100 | 2.7 |
| sample C7 | La$_{0.9}$Sr$_{0.1}$AlO$_3$ | solid phase reaction | 90 | 3.8 |
| sample C8 | LaGaO$_3$ | solid phase reaction | 100 | 6.4 |
| sample C9 | BaZrO$_3$ | solid phase reaction | 0 | 1.7 |
| sample C10 | SrTiO$_3$ | complex polymerization | 0 | 2.5 |
| sample C11 | Sr$_{0.8}$La$_{0.2}$TiO$_3$ | complex polymerization | 20 | 0.2 |
| sample C12 | BaZr$_{0.9}$Y$_{0.1}$O$_3$ | complex polymerization | 0 | 0.5 |
| sample C13 | BaZrO$_3$ | complex polymerization | 0 | 0.1 |

## INTERNATIONAL SEARCH REPORT

| International application No. |
|---|
| **PCT/JP2023/045471** |

| **A.** | **CLASSIFICATION OF SUBJECT MATTER** |
|---|---|

*B01J 23/10*(2006.01)i; *C07B 35/04*(2006.01)i; *C07C 2/84*(2006.01)i; *C07C 11/04*(2006.01)i; *C07B 61/00*(2006.01)n
FI:   B01J23/10 Z; C07B35/04; C07C2/84; C07C11/04; C07B61/00 300

According to International Patent Classification (IPC) or to both national classification and IPC

| **B.** | **FIELDS SEARCHED** |
|---|---|

Minimum documentation searched (classification system followed by classification symbols)

B01J21/00-38/74; C07B61/00; C07C2/84; C07C11/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | SIM, Yujin et al. Preparation of LaAlO3 perovskite catalysts by simple solid-state method for oxidative coupling of methane. Catalysis Today. 04 November 2019, vol. 352, pp. 134-139, DOI: 10.1016/j.cattod.2019.10.038<br>abstract, p. 134, left column, 1st paragraph to p. 135, right column, 3rd paragraph, p. 138, right column, 2nd paragraph to p. 139, left column, 1st paragraph, fig. 1, 3, 5, 7 | 8-15 |
| X | GAN, Rongguang et al. Effect of B Site Substitution on the Catalytic Activity of La-Based Perovskite for Oxidative Coupling of Methane. physica status solidi (b). 30 January 2022, vol. 259, no. 9, 2100544, DOI: 10.1002/pssb.202100544<br>abstract, p. 1, left column, 1st paragraph to p. 4, left column, 1st paragraph, p. 5, right column, 4th paragraph to p. 6, right column, 1st paragraph, fig. 3, 5 | 8-15 |
| X | TANAKA, Keisuke et al. Oxidative Coupling of Methane over Ba-incorporated LaInO3 Perovskite Catalyst. Journal of the Japan Petroleum Institute. 2012, vol. 55, no. 1, pp. 71-72, DOI: 10.1627/jpi.55.71<br>abstract, p. 71, left column, 1st paragraph to p. 72, left column, 1st paragraph, tables 1-2 | 13-15 |

☑ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **16 February 2024** | **05 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
| --- |
| **PCT/JP2023/045471** |

## C. DOCUMENTS CONSIDERED TO BE RELEVANT

| Category* | Citation of document, with indication, where appropriate, of the relevant passages | Relevant to claim No. |
| --- | --- | --- |
| X | SPINICCI, R. et al. Oxidative coupling of methane on LaAlO3 perovskites partially substituted with alkali or alkali-earth ions. Journal of Molecular Catalysis A: Chemical. November 2001, vol. 176, no. 1-2, pp. 253-265, DOI: 10.1016/S1381-1169(01)00265-5 abstract, p. 253, left column, 1st paragraph to p. 255, right column, 2nd paragraph, p. 256, left column, 2nd paragraph, p. 264, right column, 3rd paragraph to p. 265, left column, 4th paragraph, table 1, fig. 7 | 13-15 |
| X | WANG, Jingyi et al. Perovskite catalysts for oxidative coupling of methane improved by Y-doping. Asia-Pacific Journal of Chemical Engineering. 14 December 2022, vol. 18, no. 2, e2863, DOI: 10.1002/apj.2863 abstract, p. 1, left column, 1st paragraph to p. 3, right column, 1st paragraph, p. 5, left column, 3rd paragraph to p. 8, right column, 1st paragraph, fig. 6 | 8-12 |
| X | CN 107008243 A (CHINA PETROLEUM & CHEMICAL CORPORATION) 04 August 2017 (2017-08-04) claims, paragraphs [0005]-[0014], example 1, fig. 1 | 8-9, 11 |
| X | CN 107876076 A (DALIAN UNIVERSITY OF TECHNOLOGY) 06 April 2018 (2018-04-06) claims, paragraphs [0005]-[0024], examples 1-6, fig. 3-4 | 8-9, 11-12 |
| A | JP 2014-509328 A (GENERAL ELECTRIC COMPANY) 17 April 2014 (2014-04-17) entire text | 1-15 |
| A | JP 2020-104092 A (KUBOTA CORPORATION) 09 July 2020 (2020-07-09) entire text, all drawings | 1-15 |
| P, A | WO 2022/270403 A1 (NGK SPARK PLUG CO., LTD.) 29 December 2022 (2022-12-29) entire text, all drawings | 1-15 |

Form PCT/ISA/210 (second sheet) (July 2022)

## INTERNATIONAL SEARCH REPORT

| International application No. |
|---|
| **PCT/JP2023/045471** |

---

**Box No. III     Observations where unity of invention is lacking (Continuation of item 3 of first sheet)**

This International Searching Authority found multiple inventions in this international application, as follows:

Document 1: SIM, Yujin et al. Preparation of LaAlO3 perovskite catalysts by simple solid-state method for oxidative coupling of methane. Catalysis Today. 04 November 2019, vol. 352, pp. 134-139, DOI: 10.1016/j.cattod.2019.10.038

Claims are classified into the following three inventions.

(Invention 1) Claims 1-7
    Claims 1-7 have the special technical feature of a
"catalyst for dehydrogenation reaction, comprising
    a main phase composed of a perovskite-type oxide represented by the general formula $(A_{1-x}A'_x)(Zr_{1-y-z}B_yB'_z)O_{3-\delta}$ (where, A is at least one element selected from the alkali earth metals, A' is at least one element selected from lanthan (La) and yttrium (Y), and B is at least one element selected from titanium (Ti) and cerium (Ce), B' is at least one element selected from yttrium (Y), scandium (Sc), ytterbium (Yb), aluminum (Al), indium (In), and neodymium (Nd), wherein $0 \leq x \leq 0.4$, $0.3 \leq (1-z) \leq 1$, $0 \leq y$, $0 < (1-y-z)$, and $\delta$ represents the amount of oxygen deficiency), and
    a secondary phase composed of at least one of the three complex oxides represented by the general formula general formula $AB'_2O_4$, $A_2B'_2O_5$, $A_3B'_4O_9$ (wherein A and B' are elements in common with A and B' comprising said perovskite-type oxide),"
and are thus classified as invention 1.

(Invention 2) Claims 8-12
    Document 1 (see, in particular, ABSTRACT, p. 134, left column, 1st paragraph to p. 135, right column, 3rd paragraph; p. 138, right column, 2nd paragraph to p. 139, left column, 1st paragraph; and fig. 1, 3, 5, 7) describes a $LaAlO_3$ perovskite catalyst for oxidative coupling of methane, the catalyst for dehydrogenation reaction that satisfies all the matters specified in claims 8 to 12, and said claims lack novelty in document 1, thus do not have a special technical feature. That is, claims 8-12 do not have a special technical feature identical or corresponding to that of claim 1 classified as invention 1.
    Also, claim 8-12 are not dependent on claim 1 and are not substantially identical to or similarly closely related to any of the claims classified as invention 1.
    Thus, claims 8-12 cannot be classified as invention 1 but are classified as invention 2.

(Invention 3) Claims 13-15
    Document 1 is described as above, (in particular, see ABSTRACT, p. 134, left column, 1st paragraph to p. 135, right column, 3rd paragraph, p. 138, right column, 2nd paragraph to p. 139, left column, 1st paragraph, fig. 1, 3 1, 7) claims 13-15 lack novelty in light of document 1, and thus do not have a special technical feature. That is, claims 13-15 do not have a special technical feature identical or corresponding to that of claim 1 classified as invention 1 and claim 8 classified as invention 2.
    Also, claims 13-15 are not dependent on claim 1 classified as invention 1 or claim 8 classified as invention 2, and are not substantially identical to or similarly closely related to any of the claims classified as invention 1 or invention 2.
    Thus, claims 13-15 cannot be classified as either invention 1 or 2 and are thus classified as invention 3.

Form PCT/ISA/210 (continuation of first sheet) (July 2022)

**EP 4 640 310 A1**

**INTERNATIONAL SEARCH REPORT**

International application No.

**PCT/JP2023/045471**

**Box No. III    Observations where unity of invention is lacking (Continuation of item 3 of first sheet)**

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## INTERNATIONAL SEARCH REPORT
### Information on patent family members

International application No.

**PCT/JP2023/045471**

| Patent document cited in search report | | | Publication date (day/month/year) | Patent family member(s) | | | Publication date (day/month/year) |
|---|---|---|---|---|---|---|---|
| CN | 107008243 | A | 04 August 2017 | (Family: none) | | | |
| CN | 107876076 | A | 06 April 2018 | (Family: none) | | | |
| JP | 2014-509328 | A | 17 April 2014 | US | 2013/0267750 | A1 | |
| | | | | entire text | | | |
| | | | | WO | 2012/087550 | A1 | |
| | | | | EP | 2655564 | A1 | |
| | | | | CN | 102557855 | A | |
| | | | | CA | 2821249 | A | |
| | | | | MX | 2013007179 | A | |
| | | | | KR | 10-2014-0017531 | A | |
| | | | | ZA | 201305077 | B | |
| | | | | BR | 112013015872 | A | |
| JP | 2020-104092 | A | 09 July 2020 | US | 2021/0346870 | A1 | |
| | | | | entire text, all drawings | | | |
| | | | | WO | 2020/137382 | A1 | |
| | | | | EP | 3903931 | A1 | |
| | | | | CN | 112334226 | A | |
| | | | | CA | 3103773 | A | |
| WO | 2022/270403 | A1 | 29 December 2022 | (Family: none) | | | |

Form PCT/ISA/210 (patent family annex) (July 2022)

**REFERENCES CITED IN THE DESCRIPTION**

**Patent documents cited in the description**

- JP 8010620 A

- JP 61282323 A

**Non-patent literature cited in the description**

- **SEOYEON LIM** ; **JAE-WOOK CHOI** ; **DONG JIN SUH** ; **KWANG HO SONG** ; **HYUNG CHUL HAM** ; **JEONG-MYEONG HA**. Combined experimental and density functional theory (DFT) studies on the catalyst design for the oxidative coupling of methane. *Journal of Catalysis*, July 2019, vol. 375, 478-492